(19) Europäisches Patentamt European Patent Office Office européen des brevets

(11) **EP 3 264 984 B1**

(12) **FASCICULE DE BREVET EUROPÉEN**

(45) Date de publication et mention de la délivrance du brevet:
**28.11.2018 Bulletin 2018/48**

(21) Numéro de dépôt: **16714986.3**

(22) Date de dépôt: **26.02.2016**

(51) Int Cl.:
*A61B 5/157* (2006.01)    *B01L 3/00* (2006.01)
*A61B 5/15* (2006.01)    *A61B 10/00* (2006.01)

(86) Numéro de dépôt international:
**PCT/FR2016/050446**

(87) Numéro de publication internationale:
**WO 2016/139409 (09.09.2016 Gazette 2016/36)**

(54) **DISPOSITIF DE PRÉLÈVEMENT D'UN ÉCHANTILLON LIQUIDE PAR CAPILLARITÉ**

VORRICHTUNG ZUM GEWINNEN EINER FLÜSSIGEN PROBE DURCH KAPILLARWIRKUNG

DEVICE FOR COLLECTING A LIQUID SAMPLE BY CAPILLARY ACTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.03.2015 FR 1551725**

(43) Date de publication de la demande:
**10.01.2018 Bulletin 2018/02**

(73) Titulaire: **Avalun**
**38000 Grenoble (FR)**

(72) Inventeurs:
• **POUTEAU, Patrick**
**38240 Meylan (FR)**
• **POHER, Vincent**
**62340 Guines (FR)**

(74) Mandataire: **GIE Innovation Competence Group**
**310, avenue Berthelot**
**69372 Lyon Cedex 08 (FR)**

(56) Documents cités:
**WO-A1-01/05505    WO-A1-2005/020817**
**WO-A1-2014/135652    DE-A1- 4 307 735**

**Description**

**DOMAINE TECHNIQUE**

Le domaine de l'invention est celui du prélèvement en vue de l'analyse d'échantillons liquide, par exemple des échantillons chimiques et/ou biologiques. L'invention porte sur un dispositif de prélèvement d'un échantillon liquide comportant un canal dans lequel l'échantillon est destiné à s'écouler par capillarité, ainsi que sur un procédé d'analyse de l'échantillon liquide prélevé au moyen d'un tel dispositif.

**ÉTAT DE LA TECHNIQUE ANTÉRIEURE**

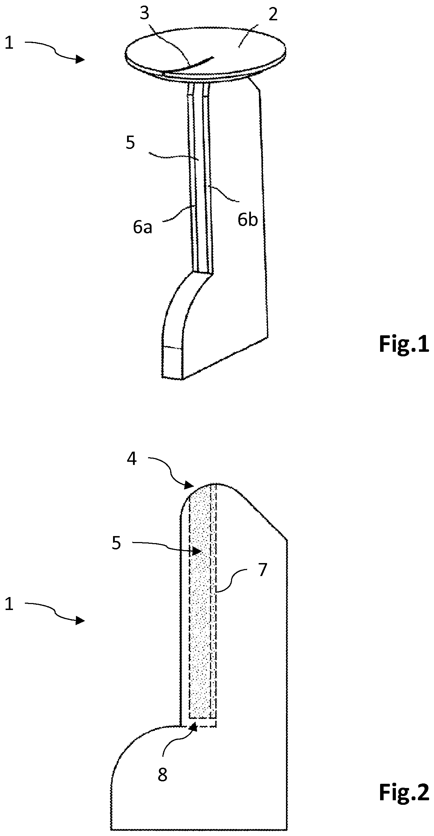

Les figures 1 et 2 illustrent un exemple de dispositif de prélèvement 1 d'un échantillon liquide par capillarité tel que décrit dans le document WO2014135652. Il comporte une surface de contact 2 au niveau de laquelle est prévue une ouverture traversante 3 qui débouche sur une extrémité d'entrée 4 d'un canal 5. Le canal, formé de deux parois latérales 6a, 6b parallèles entre elles et reliées l'une à l'autre par une paroi de fond 7, s'étend longitudinalement de l'extrémité d'entrée 4 jusqu'à une deuxième extrémité 8.

La paroi de fond 7 est agencée de sorte que la distance entre les deux parois latérales 6a, 6b diminue en direction du fond du canal. Ainsi, lorsqu'un échantillon liquide est déposé sur la surface de contact 2, il entre dans le canal 5 au niveau de l'extrémité d'entrée 4 puis s'écoule par capillarité le long du canal 5 jusqu'à la deuxième extrémité 8. La force motrice est une force capillaire dont l'intensité est augmentée par le rapprochement des parois latérales 6a, 6b au niveau du fond. Pour stopper l'écoulement du liquide au niveau de la deuxième extrémité 8 du canal, un moyen d'arrêt d'écoulement, tel qu'une zone hydrophobe ou un élargissement brusque des dimensions du canal, est prévu.

Le canal comporte habituellement une portion, dite chambre de mesure ou d'analyse, dans laquelle sont prévues par exemple des réactifs séchés ou lyophilisés ou des électrodes. Les parois latérales du canal étant généralement transparentes au rayonnement visible ou infrarouge, une mesure optique peut être réalisée pour l'analyse chimique ou biologique de l'échantillon liquide ou de la réaction chimique initiée lors de la reprise du réactif séché ou lyophilisé par l'échantillon.

Le canal présente ici une section transversale ouverte, c'est-à-dire que le canal 5 est ouvert sur l'environnement sur toute sa partie longitudinale opposée à la paroi de fond 7.

Un inconvénient de ce dispositif est alors que les surfaces internes du canal sont difficiles d'accès, notamment lorsqu'on souhaite les fonctionnaliser par le dépôt de réactifs séchés ou lyophilisés ou par la mise en place d'électrodes ou par un traitement chimique ou biologique localisé sur la surface. A titre d'exemple, dans le cas de la fonctionnalisation de la chambre d'analyse par des réactifs séchés ou lyophilisés, la difficulté d'accès à la chambre d'analyse rend délicat et peu reproductible le dépôt et le séchage des réactifs. Il est en effet nécessaire d'introduire un solvant contenant les réactifs au niveau de l'extrémité d'entrée du canal pour qu'il s'écoule jusqu'à la chambre d'analyse, avant de procéder à l'évaporation ou à la sublimation du solvant.

De plus, l'intérieur du canal est accessible de l'extérieur sur toute sa partie longitudinale ouverte, ce qui augmente le risque de pollution du canal par un élément extérieur, et peut nécessiter de structurer l'intérieur du canal de manière à former des moyens d'ancrage du liquide destinés à confiner l'échantillon liquide à l'intérieur du canal et éviter ainsi une contamination du milieu extérieur par le liquide.

**EXPOSÉ DE L'INVENTION**

L'invention a pour objectif de remédier au moins en partie aux inconvénients de l'art antérieur, et plus particulièrement de proposer un dispositif de prélèvement d'échantillon liquide par capillarité permettant à la fois un accès facilité aux surfaces internes du canal et un confinement efficace du canal.

L'invention propose à cet effet un dispositif de prélèvement d'un échantillon liquide par capillarité de prélèvement d'un échantillon liquide par capillarité, comportant :

- un premier élément, comprenant une partie mâle,

    ◦ la partie mâle comportant au moins un canal à section transversale ouverte, s'étendant longitudinalement entre une première extrémité d'entrée et une seconde extrémité, le canal étant formé par une paroi longitudinale de fond de canal bordée de deux parois latérales formant les flancs du canal ;

- un second élément, distinct du premier élément, comprenant une partie femelle,

    ◦ la partie femelle comportant une paroi périphérique qui délimite transversalement une cavité destinée à loger la partie mâle ;
    ◦ une partie de la paroi périphérique étant destinée, lorsque la partie femelle loge la partie mâle, à former un capot fermant la section transversale du canal.

De préférence, au moins un des flancs du canal est agencé de sorte que, lorsque la partie femelle loge la partie mâle, la distance, dans un plan transversal à l'axe longitudinal du canal, entre deux parois du canal diminue en direction d'une bordure transversale du canal.

La partie mâle peut être formée d'une plaque de section transversale sensiblement rectangulaire, le canal étant disposé au niveau d'une face longitudinale dite supérieure de la plaque formant la partie mâle.

Le canal peut comporter au moins une portion dite chambre d'analyse, disposée au niveau de la face supérieure de la partie mâle ou au niveau d'une face dite inférieure, opposée à la face supérieure, de la partie mâle.

Selon un mode de réalisation, le canal comporte au moins une chambre d'analyse, à section transversale ouverte, disposée au niveau de la face inférieure de la partie mâle, communiquant avec la portion du canal située au niveau de la face supérieure, et dans lequel une partie de la paroi périphérique de la partie femelle est destinée, lorsque la partie femelle loge la partie mâle, à former un second capot fermant la section transversale de ladite chambre d'analyse.

Ladite chambre d'analyse peut communiquer avec la portion du canal située au niveau de la face supérieure par un conduit débouchant au niveau d'une zone centrale de ladite chambre d'analyse.

De préférence, les dimensions de la partie mâle et celles de la partie femelle définissant la cavité sont choisies de sorte que, lorsque la partie femelle loge la partie mâle, le capot appuie sur la face supérieure de la plaque de la partie mâle ou inversement, et éventuellement sur la face inférieure opposée à la face supérieure, de manière à assurer la fermeture hermétique de la section transversale du canal.

Avantageusement, au moins une portion de la surface de la paroi longitudinale de fond, au moins une portion des flancs du canal et/ou au moins une portion de la surface du capot présente(nt) une mouillabilité différente de celle des autres surfaces du canal.

Le second élément peut comporter une surface de contact destinée à recevoir l'échantillon liquide, assemblée à la partie femelle, de sorte qu'une ouverture de collecte de la cavité débouche au niveau de la surface de contact, et dans lequel, lorsque la partie femelle loge à la partie mâle, une paroi transversale d'extrémité de la partie mâle au niveau de laquelle se situe la première extrémité d'entrée du canal, affleure la surface de contact.

Avantageusement, la seconde extrémité du canal communique avec un évent débouchant sur l'environnement du dispositif.

Avantageusement, le premier élément comporte un talon de préhension sur lequel est assemblée la partie mâle.

Le talon peut être formé d'une plaque qui s'étend dans le plan de la partie mâle, et comporte au moins une portion en saillie vis-à-vis de la plaque du talon.

Le talon peut comporter une surface destinée à former une butée vis-à-vis de la paroi périphérique de la partie femelle lorsque celle-ci loge la partie mâle.

Selon un mode de réalisation, le canal comprend au moins une portion formant une chambre d'analyse comportant des réactifs séchés ou lyophilisés, au moins une électrode, ou une membrane absorbante.

Le canal peut être délimité transversalement par des parois latérales de délimitation destinées à être en contact avec le capot, ces dernières séparant le canal d'une zone périphérique qui entoure au moins en partie le canal, le canal comportant une surface dite de fond en regard du capot et la zone périphérique comportant une surface dite périphérique en regard de la paroi périphérique, une distance entre la surface périphérique et la paroi périphérique en regard, suivant un axe orthogonal à la surface de fond, étant supérieure à une distance entre la surface de fond et le capot en regard.

L'invention porte également sur un procédé de réalisation d'un échantillon liquide à l'aide d'un dispositif de prélèvement selon l'une quelconque des caractéristiques précédentes, comportant les étapes dans lesquelles :

-   on réalise un premier élément comprenant une partie mâle, celle-ci comportant au moins un canal à section transversale ouverte, s'étendant longitudinalement entre une première extrémité d'entrée et une seconde extrémité, le canal étant formé par une paroi longitudinale de fond de canal bordée de deux parois latérales formant les flancs du canal ;
-   on réalise un second élément distinct du premier élément, comprenant une partie femelle, celle-ci comportant une paroi périphérique qui délimite transversalement une cavité destinée à loger la partie mâle, une partie de la paroi périphérique étant destinée, lorsque la partie femelle loge la partie mâle, à former un capot fermant la section transversale du canal ;
-   on fonctionnalise une zone du canal formant une chambre d'analyse ;
-   on introduit la partie mâle dans la partie femelle.

**BRÈVE DESCRIPTION DES DESSINS**

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels, outre les figures 1 et 2 déjà décrites précédemment :

la figure 3 est une vue en perspective d'un dispositif de prélèvement d'un échantillon liquide selon un mode de réalisation, où la partie mâle est désengagée de la partie femelle ;
la figure 4 est une vue en perspective du premier élément du dispositif de prélèvement représenté sur la figure 3 ;
les figures 5 et 6 sont des vues schématiques en coupe transversale du canal du dispositif de prélèvement selon deux modes de réalisation ;

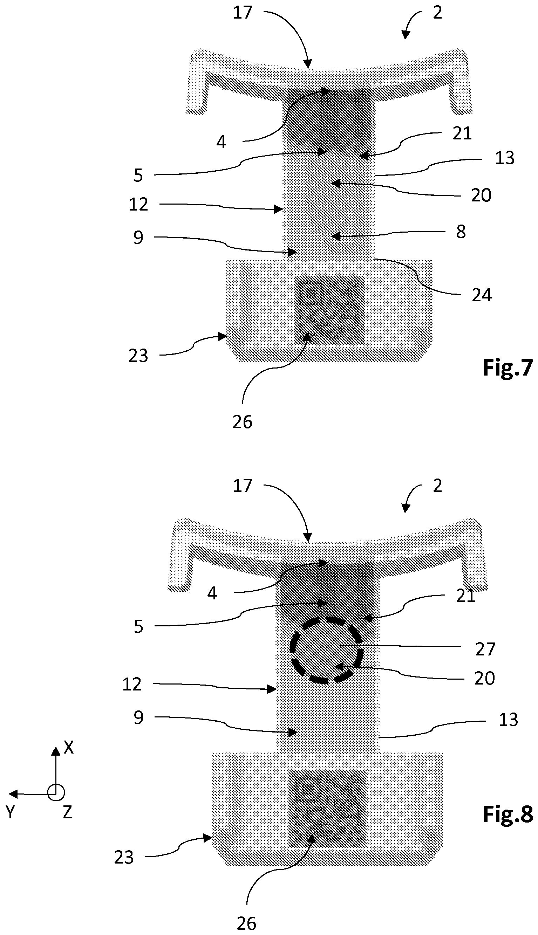

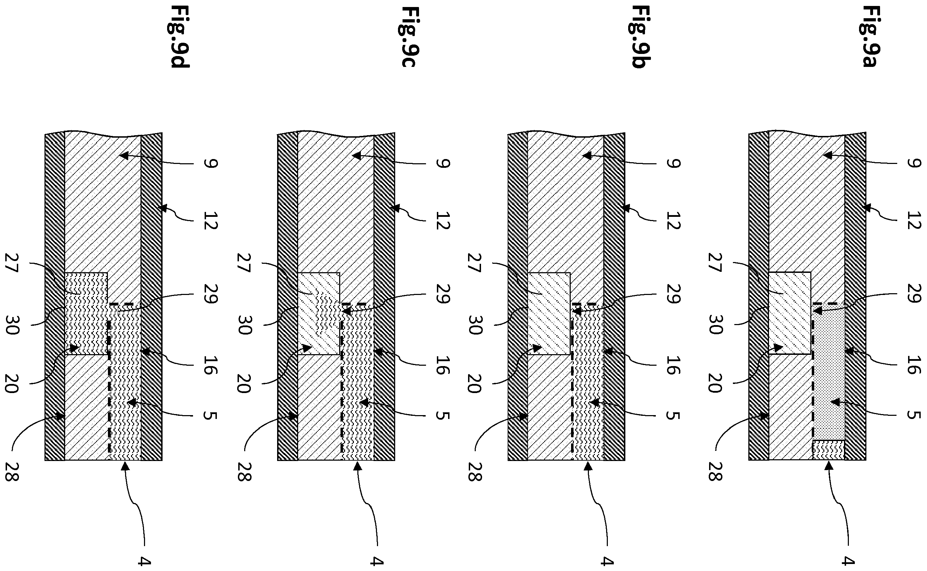

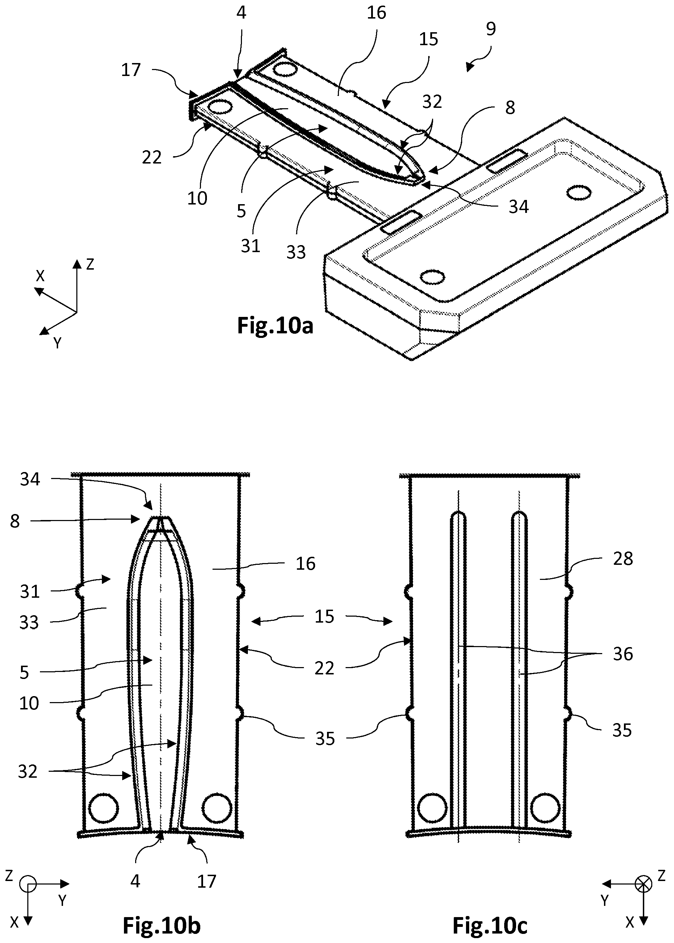

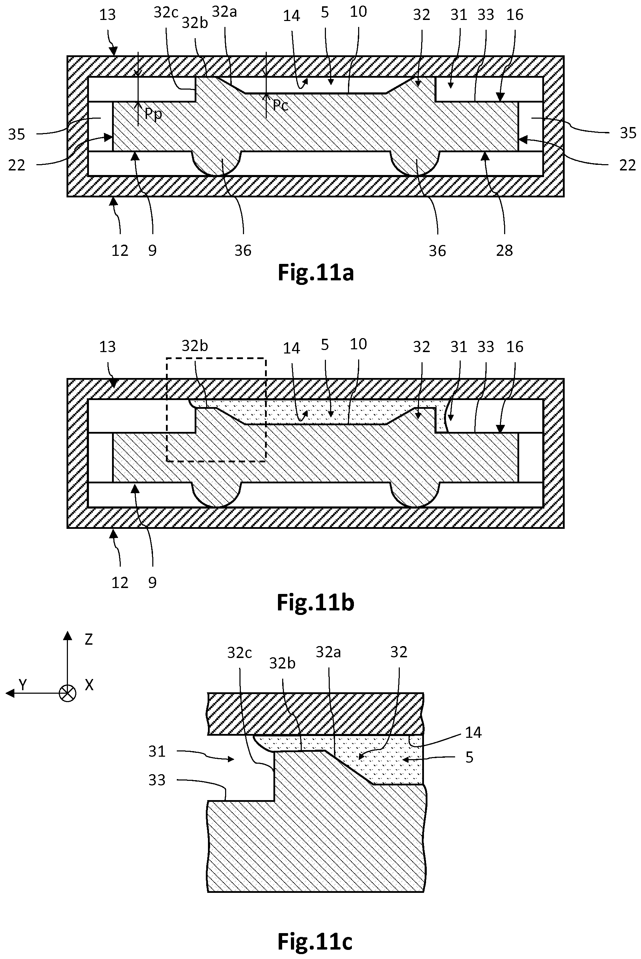

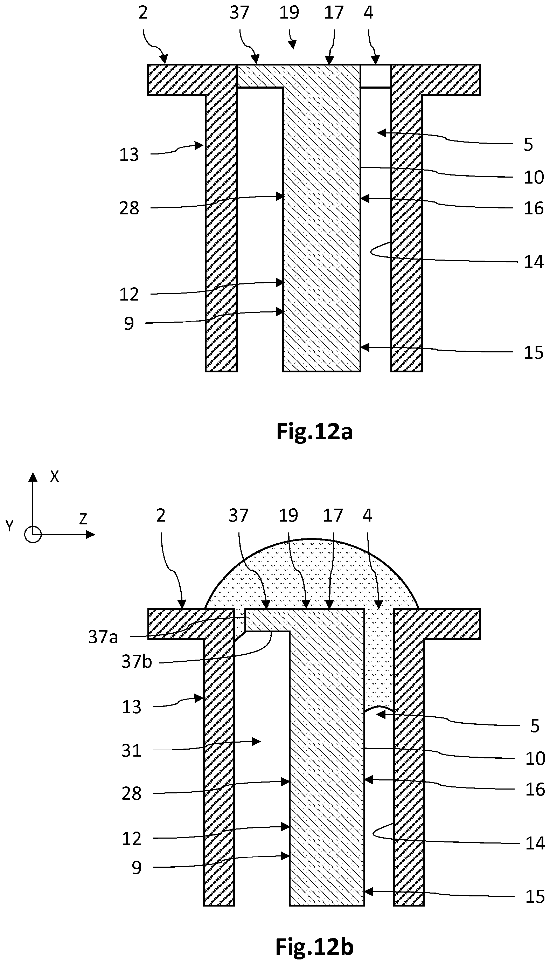

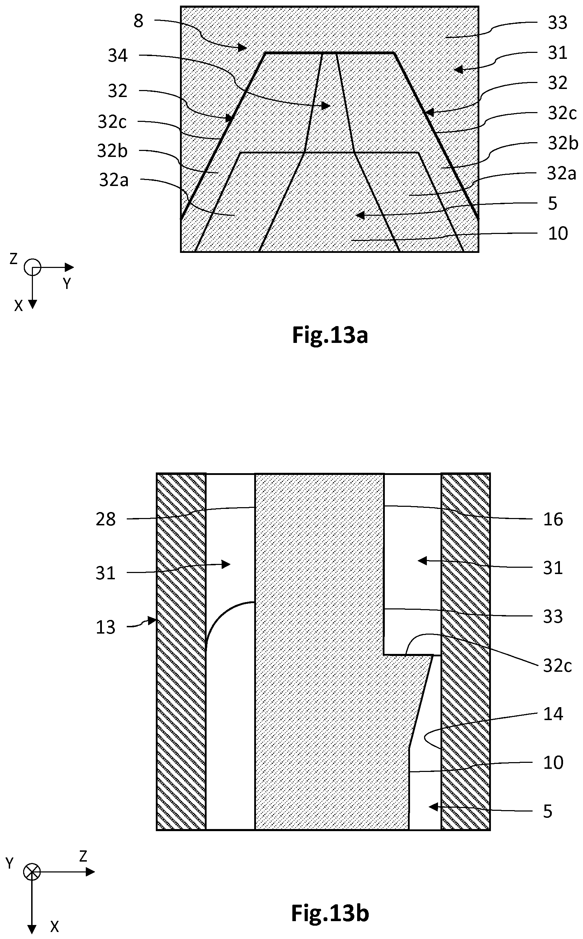

la figure 7 est une vue de face d'un dispositif de prélèvement dont la partie mâle est introduite dans la partie femelle, selon un mode de réalisation où l'évent débouche au niveau de la surface de contact ; la figure 8 est une vue de face d'un dispositif de prélèvement dont la partie mâle est introduite dans la partie femelle, selon un mode de réalisation où une chambre d'analyse du canal comporte une membrane absorbante ; les figures 9a à 9d sont des représentations schématiques, en coupe longitudinale, d'une partie du dispositif de prélèvement représenté sur la figure 8, pour différents moments de l'écoulement de l'échantillon liquide dans le canal ; les figures 10a, 10b et 10c sont des vues, respectivement en perspective, de dessus et de dessous, d'une partie mâle suivant un autre mode de réalisation où le canal est entouré au moins en partie par une zone périphérique de plus grande profondeur ; les figures 11a à 11c sont des vues schématiques en coupe transversale du dispositif de collecte lorsque la partie femelle loge la partie mâle selon cet autre mode de réalisation ; les figures 12a et 12b sont des vues schématiques en coupe longitudinale du dispositif de collecte au niveau de l'entrée du canal ; les figures 13a et 13b sont des vues schématiques de l'évent du canal, en vue de dessus (fig.13a) et en coupe longitudinale (fig.13b).

## EXPOSÉ DÉTAILLÉ DE MODES DE RÉALISATION PARTICULIERS

Sur les figures et dans la suite de la description, les mêmes références représentent les éléments identiques ou similaires.

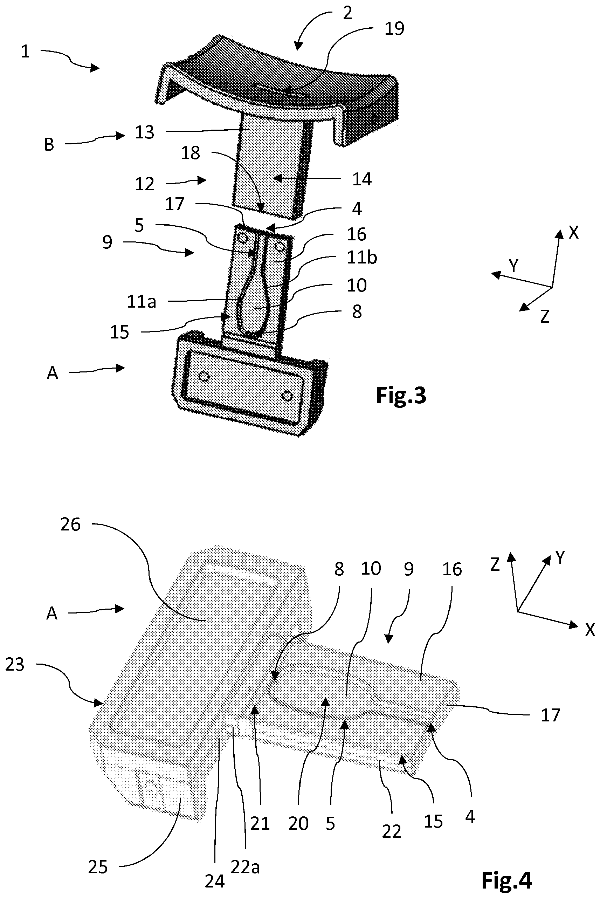

La figure 3 illustre un dispositif de prélèvement 1 d'échantillon liquide par capillarité, selon un mode de réalisation.

Le dispositif de prélèvement comporte deux éléments A, B, distincts l'un de l'autre, et destinés à coopérer l'un avec l'autre pour rendre le dispositif opérationnel.

Le premier élément A comprend une partie mâle 9 au niveau de laquelle se situe un canal 5 à section transversale ouverte. Le canal 5 s'étend longitudinalement entre une première extrémité 4, dite d'entrée, apte à recevoir un échantillon liquide, et une seconde extrémité 8. Le canal 5 est formé par une paroi longitudinale 10, appelée dans la suite de la description paroi de fond, bordée de deux parois latérales 11a, 11b qui forment les flancs du canal 5.

Le second élément B comporte une partie femelle 12 formée d'une paroi périphérique 13 qui délimite transversalement une cavité destinée à loger, ou recevoir, la partie mâle 9. De plus, une partie de la paroi périphérique 13 est destinée, lorsque la partie femelle loge la partie mâle, à former un capot 14 fermant la section transversale du canal 5.

Ainsi, pour rendre opérationnel le dispositif de prélèvement 1 d'un échantillon liquide, la partie mâle 9 du premier élément A est introduite dans la partie femelle 12 du second élément B, de sorte que la paroi périphérique 13 ferme la section transversale du canal 5, de préférence sur toute la longueur du canal. Un échantillon liquide peut alors être amené au contact de l'extrémité d'entrée 4 du canal 5, ce qui provoque son insertion dans le canal 5 et l'écoulement capillaire de l'échantillon le long du canal 5 en direction de la deuxième extrémité 8.

Il résulte de cette réalisation en deux éléments distincts A et B du dispositif de prélèvement 1 dont le canal 5 présente une section transversale ouverte au niveau longitudinal :

- avant insertion de la partie mâle dans la partie femelle, un accès direct à l'intérieur du canal ;
- après insertion de la partie mâle dans la partie femelle, un meilleur confinement du canal.

L'accès direct à l'intérieur du canal, c'est-à-dire à son volume interne et à au moins une partie de ses surfaces internes, permet de fonctionnaliser et/ou traiter l'intérieur du canal, en partie ou totalement, avec une simplicité et une efficacité que l'exemple de l'art antérieur ne permet pas d'atteindre.

Ainsi, dans le cas où on souhaite déposer dans le canal un réactif séché ou lyophilisé destiné à interagir avec l'échantillon liquide, l'accès direct à l'intérieur du canal permet de procéder de manière simple et rapide à un dépôt particulièrement homogène du réactif. En effet, l'intérieur du canal est accessible directement sur toute sa longueur, alors que dans l'exemple de l'art antérieur le canal n'est accessible qu'à partir de son extrémité d'entrée. La précision de positionnement des outils de dépôt du ou des réactifs à sécher ou lyophiliser peut alors être simplifiée. Il est également possible de réaliser simplement un dépôt localisé du ou des réactifs à sécher ou lyophiliser, directement dans une zone désirée du canal (que ce soit à l'entrée, au milieu ou à la sortie du canal). L'exemple de l'art antérieur n'offre pas une telle simplicité dans le positionnement localisé du ou des réactifs.

Il est également possible de déposer dans le canal, de manière simple et précise, des électrodes ou une membrane absorbante, voire de fonctionnaliser par liaison chimique l'intérieur du canal, par exemple par immobilisation locale d'une ou plusieurs molécules chimiques ou entités biologiques (protéine, séquence ADN, anticorps, etc...) directement sur une paroi du canal, par exemple par liaison chimique covalente.

Le dispositif de prélèvement offre également, lorsque la partie femelle loge la partie mâle, un confinement particulièrement efficace du canal, ce qui permet de limiter, voire éviter, la contamination de l'environnement extérieur par le liquide présent dans le canal ainsi que la pollution du canal à partir de l'extérieur.

Dans l'exemple de la figure 3, la partie mâle 9 est formée d'une plaque 15 présentant une section transversale sensiblement rectangulaire. Le canal 5 est disposé au niveau d'une face longitudinale 16, dite supérieure, de la plaque, de sorte que l'extrémité d'entrée 4 du canal 5 débouche sur une paroi transversale d'extrémité 17 de la plaque 15. La plaque 15 présente ici une section transversale rectangulaire, mais tout type de section peut également convenir, par exemple carrée, voire également circulaire.

On définit un repère orthonormé tridimensionnel tel qu'illustré sur la figure 3, où l'axe X est orienté suivant l'axe longitudinal de la partie mâle 9, l'axe Y suivant sa largeur et l'axe Z suivant son épaisseur. On définit ici la largeur du canal 5 comme la distance, suivant l'axe Y, entre deux parois du canal 5, et la profondeur du canal 5 comme la distance, suivant l'axe Z, entre deux parois du canal 5.

La paroi longitudinale de fond 10 du canal 5, présente avantageusement une dimension, en coupe transversale, supérieure à celle de chacun des flancs 11a, 11b. En d'autres termes, le facteur de forme transversal du canal 5, à savoir le rapport entre la largeur du canal sur sa profondeur, est, dans cet exemple, strictement supérieur à 1, de préférence supérieur à 5, voire à 10.

Dans l'exemple de la figure 3, la paroi périphérique 13 de la partie femelle 12 s'étend longitudinalement, suivant l'axe X, de manière à former une cavité apte à recevoir et loger, de préférence entièrement, la partie mâle 9. De plus, les dimensions internes de la paroi périphérique 13 sont ajustées de sorte que la surface du pourtour de la partie mâle 9 vienne au contact de la surface interne de la paroi périphérique 13, lorsque la partie femelle 12 loge la partie mâle 9.

Dans cet exemple, la paroi périphérique 13 forme une cavité de section transversale, dans le plan (Y,Z), sensiblement rectangulaire, correspondant à la section transversale rectangulaire de la partie mâle 9. Cependant, toute autre forme, complémentaire de celle de la partie mâle, peut convenir.

Dans un mode de réalisation particulier, la section transversale de la paroi périphérique 13 présente, suivant la direction de l'axe X, une diminution homothétique de ces dimensions, ainsi que la section transversale de la partie mâle 9. Ceci permet un emboitement de type cône-cône facilitant l'assemblage des deux éléments du dispositif. La cavité s'étend entre une ouverture d'insertion 18, par laquelle la partie mâle est destinée à être introduite, et une ouverture opposée 19, dite de collecte, apte à recevoir l'échantillon liquide pour l'insertion de celui-ci dans le canal.

Le second élément B comporte en outre une surface de contact 2, ici sous forme d'une coupelle, destinée à recevoir l'échantillon liquide. La partie femelle 12 est assemblée à la surface de contact 2, de sorte que l'ouverture de collecte 19 de la cavité débouche au niveau de la surface de contact 2. La surface de contact 2 s'étend de manière sensiblement orthogonale à la partie femelle 12, et peut présenter une forme courbe, évasée, plus précisément convexe, pour permettre à faciliter la mise en contact de l'échantillon liquide avec l'ouverture de collecte 19. Cette courbe est aussi particulièrement adaptée à la dépose d'une goutte pendante au bout d'un doigt humain, tel qu'elle peut se présenter lors d'un prélèvement de sang capillaire au bout d'un doigt.

Ainsi, lorsqu'un échantillon liquide est déposé sur la surface de contact 2 et vient au contact de l'ouverture de collecte 19, il s'introduit par capillarité dans le canal 5 par son extrémité d'entrée 4. A ce titre, lorsque la surface de contact 2 présente une forme évasée, comme représentée sur la figure 3, il est avantageux que la paroi transversale d'extrémité 17 de la partie mâle 9 présente également une forme courbe de même rayon de courbure. Ainsi, la surface de contact 2 et la surface de la paroi transversale d'extrémité 17 de la paroi mâle 9 présentent ensemble une surface sensiblement continue, sans aspérité en saillie ou en retrait, hormis l'ouverture de l'extrémité d'entrée du canal.

Pour favoriser l'introduction de l'échantillon liquide par capillarité dans le canal 5, les parois du dispositif de prélèvement présente idéalement un angle de mouillage inférieur à 90° et idéalement inférieur à 50°. Ceci peut être obtenu en choisissant des matériaux présentant naturellement cet angle de mouillage ou bien par traitement chimique après fabrication des éléments A et B du dispositif. Un traitement au plasma oxygène ou bien un traitement par insolation avec une lampe UV intense en présence d'oxygène peut être aussi utilisé.

La figure 4 est une vue détaillée et en perspective du premier élément selon le mode de réalisation représenté sur la figure 3.

Le canal 5, disposé au niveau de la face supérieure 16 de la partie mâle 9, s'étend entre l'extrémité d'entrée 4 et la seconde extrémité 8. Le canal 5 comprend ici une première portion qui s'étend à partir de l'extrémité d'entrée 4 et une deuxième portion 20 qui s'étend jusqu'à la deuxième extrémité 8 du canal. La deuxième portion 20 présente un élargissement transversal, c'est-à-dire une augmentation de sa largeur suivant l'axe Y. Cette deuxième portion 20 forme une chambre d'analyse, ou de mesure, destinée à permettre l'analyse de l'échantillon liquide par des moyens appropriés présents dans la chambre.

Au niveau de la seconde extrémité 8, le canal 5 communique avec un évent 21 qui permet la mise à l'air libre du canal. L'évent 21 s'étend jusqu'à une bordure de la plaque formant la partie mâle 9. Dans cet exemple, l'évent 21 se présente sous la forme d'une rainure pratiquée dans la face supérieure 16 de la partie mâle 9, et s'étend suivant l'axe Y de manière à rejoindre les deux bordures longitudinales 22 de la partie mâle. Lorsque la partie femelle 12 loge la partie mâle 9, la paroi périphérique 13 de la partie femelle 12 comporte au moins une ouverture débouchante (non représentée) qui communique avec l'évent de manière à permettre la mise à l'air libre du canal.

Le canal 5 comporte, au niveau de sa deuxième extrémité 8, un moyen d'arrêt d'écoulement du liquide.

Ce moyen d'arrêt peut se présenter sous la forme d'une zone où au moins une partie des surfaces internes du canal sont hydrophobes vis-à-vis de l'échantillon liquide. Cette propriété hydrophobe peut être obtenue par un traitement de surface localisé ou par le dépôt d'un revêtement hydrophobe. Elle peut concerner tout ou partie des flancs, capot et fond du canal. Il peut également se présenter, comme dans le cas de la figure 4, sous la forme d'un élargissement des dimensions transversales du canal, par exemple sa largeur suivant l'axe Y, tel que décrit dans la publication d'A. Glière et C. Delattre, Modeling and fabrication of capillary stop valves for planar microfluidic systems, Sens. Actuators A, 130-131 (2006), 601-608. Cet élargissement permet de diminuer la force capillaire de mouillabilité responsable de l'écoulement du liquide. Le moyen d'arrêt peut également être une paroi de fermeture du canal, qui s'étend dans une section transversale du canal et bloque l'écoulement dans le sens longitudinal du canal.

Les dimensions de la partie mâle 9 et celles de la cavité de la partie femelle 12 sont telles que, lorsque la partie femelle loge la partie mâle, le maintien des parties mâle 9 et femelle 12 l'une dans l'autre est assuré par frottement des surfaces. Dans l'exemple de la figure 4, la partie mâle 9 comporte une partie de ses bordures longitudinales 22 dont la largeur suivant l'axe Z, et éventuellement suivant l'axe Y, augmente à mesure qu'on s'éloigne de l'extrémité d'entrée 4 du canal. Ainsi, lorsque la partie femelle loge la partie mâle, la paroi périphérique 13 de la partie femelle 12 exerce une force de pression sur ces parties latérales élargies 22a de la partie mâle 9 et sera ainsi maintenue par frottement au niveau de celles-ci.

Par ailleurs, les dimensions de la partie mâle 9 et celles de la cavité de la partie femelle 12 sont telles que, lorsque la partie femelle loge la partie mâle, le capot 14 appuie sur la face supérieure 16 de la partie mâle 9, ou inversement, de manière à assurer la fermeture hermétique de la section transversale du canal 5.

Le premier élément A comporte en outre un talon 23 dit de préhension sur lequel est fixée la partie mâle 9. Le talon 23 se présente sous la forme d'une plaque qui s'étend principalement dans le plan (X,Y) de la partie mâle 9, suivant un axe longitudinal Y sensiblement orthogonal à l'axe longitudinal X de la partie mâle 9. Il permet à un utilisateur de manipuler aisément le dispositif.

Le talon 23 comporte, de part et d'autre de la partie mâle, une surface destinée à former une butée 24 vis-à-vis de la paroi périphérique 13 de la partie femelle 12, lorsque la partie mâle est introduite dans la partie femelle. Les dimensions longitudinales de la partie mâle 9 et de la partie femelle 12 sont sensiblement égales, de sorte que, lorsque la partie femelle loge la partie mâle entièrement, l'extrémité de la paroi périphérique 13 délimitant l'ouverture d'insertion 18 est au contact de la butée 24 du talon 23 et la paroi transversale d'extrémité 17 de la partie mâle 9 se trouve au niveau de l'ouverture de collecte 19 de la cavité, l'extrémité d'entrée 4 du canal 5 affleurant la surface de contact 2.

Le talon 23 comporte également au moins une portion 25 en saillie, ici deux portions, qui s'étendent transversalement au plan (Y, Z) du talon 23. Ces portions 25 assurent une fonction de détrompeur permettant d'éviter toute erreur dans l'utilisation ou la manipulation du dispositif de prélèvement, notamment lorsqu'il s'agit de placer correctement le dispositif dans un support d'un système d'analyse.

Par ailleurs, comme l'illustrent les figures 7 et 8, le talon 23 comporte une zone 26 d'inscription d'informations au niveau de laquelle peuvent être inscrites des informations sous la forme, par exemple, d'un code Q.R (ou code *datamatrix*), permettant par exemple l'identification du type de réactif déposé dans la chambre d'analyse 20 du canal 5 et /ou une date de péremption ou de fabrication.

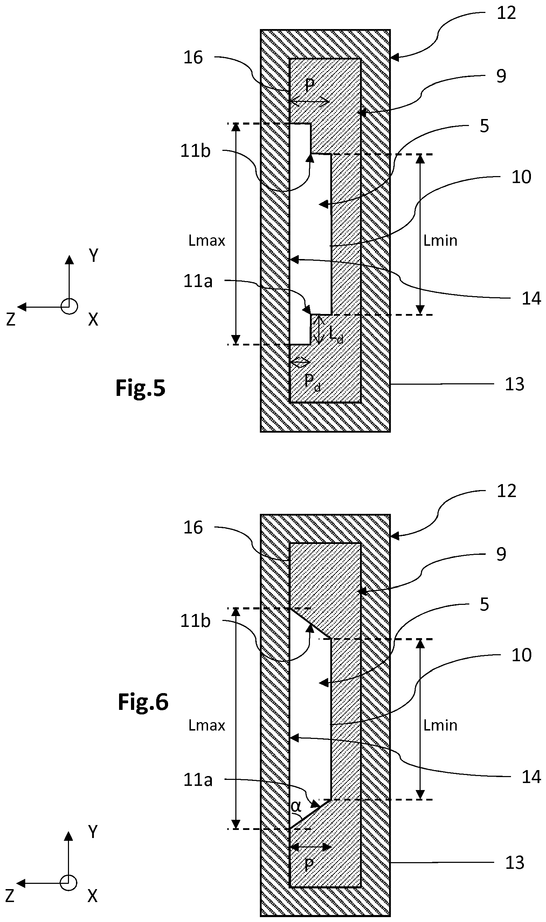

Les figures 5 et 6 sont des vues schématiques du canal, en coupe transversale, lorsque la partie femelle loge la partie mâle, selon deux variantes de réalisation.

Le repère orthonormé tridimensionnel défini en figure 3 est reproduit ici. L'axe X est orienté suivant la longueur de la partie mâle, l'axe Y suivant sa largeur et l'axe Z suivant son épaisseur. On rappelle que la largeur du canal est définie comme la distance, suivant l'axe Y, entre deux parois du canal, et la profondeur du canal comme la distance, suivant l'axe Z, entre deux parois du canal. Les bordures transversales du canal sont les extrémités de celui-ci dans le plan (Y, Z) suivant l'axe Y.

La partie mâle 9 présente une section transversale ouverte dans le plan (Y, Z) formée d'une paroi de fond 10 bordée en ses deux extrémités longitudinales par des parois latérales 11a, 11b formant des flancs de canal. La partie femelle 12 comporte une partie de sa paroi périphérique 13 qui forme un capot 14, la face interne de celui-ci venant au contact de la face supérieure 16 de la partie mâle 9 de manière à fermer la section transversale du canal 5.

De manière à augmenter l'intensité de la force capillaire qui assure l'écoulement de l'échantillon liquide dans le canal, il est avantageux d'agencer, c'est-à-dire incliner ou structurer, au moins l'un des flancs 11a, 11b du canal 5 de sorte que, lorsque la partie femelle loge la partie mâle, la distance, dans un plan transversal (Y, Z) du canal, entre deux parois du canal, diminue en direction d'une bordure transversale, suivant l'axe Y, du canal. En d'autres termes, la profondeur du canal diminue au niveau d'une des bordures du canal, de préférence sur toute la longueur du canal jusqu'à la deuxième extrémité. Ainsi, on forme un amincissement local du canal au niveau d'au moins une bordure du canal apte à générer une force capillaire de plus forte intensité, ce qui conduit à une vitesse d'écoulement du liquide plus importante.

La figure 5 illustre un mode de réalisation dans lequel le capot 14 et la paroi de fond 10 sont planes et parallèles entre elles. Les flancs 11a, 11b sont structurés de manière à former un décrochement venant au contact du capot 14. Ainsi, au niveau du décrochement, la profondeur $P_d$ du canal, donc ici la distance suivant l'axe Z entre le capot 14 et la partie du flanc parallèle au capot 14, est inférieure à la profondeur centrale P mesurée entre le capot 14 et la paroi de fond 10. Un amincissement local est donc réalisé au niveau des bordures du canal, propice à permettre un écoulement plus rapide de l'échantillon liquide dans le canal, comme le décrit le document WO2014/135652 cité précédemment.

De préférence, pour générer un écoulement capillaire spontané, les dimensions du décrochement vérifient la condition suivante :

$$L_d > P_d/2 \cdot (1/\cos\theta - 1)$$

où $L_d$ est la largeur du décrochement suivant l'axe Y, c'est-à-dire la distance entre le coude du décrochement et le fond de celui-ci, $P_d$ la profondeur du canal au niveau du décrochement, et $\theta$ l'angle de mouillage que forme le liquide sur la surface interne du canal, au niveau du point triple. On suppose ici que l'angle de mouillage est le même pour toutes les parois du canal. A titre d'exemple, la profondeur $P_d$ du décrochement est inférieure ou égale à 1mm, voire à $500\mu m$ et est de préférence comprise entre $30\mu m$ et $500\mu m$, voire entre $100\mu m$ et $200\mu m$. La largeur $L_d$ est de préférence inférieure à quelques millimètres, par exemple inférieure à 1mm, voire inférieure à 0,7mm, et notamment comprise entre $200\mu m$ et $500\mu m$.

La figure 6 illustre un autre mode de réalisation de la forme transversale du canal, en variante au mode de réalisation de la figure 5, qui diffère de celui-ci uniquement par l'agencement des flancs 11a, 11b du canal 5. Dans cet exemple, les flancs 11a, 11b sont sensiblement plans et inclinés de manière à former un angle aigu, inférieur à 90°, avec le capot 14. L'amincissement localisé du canal 5 se présente ici sous forme d'un V au niveau des bordures. La profondeur en bordure du canal est alors définie par la distance suivant l'axe Z entre le capot 14 et le flanc 11a, 11b considéré.

De préférence, pour obtenir un écoulement capillaire issu d'un effet Concus-Finn, l'inclinaison des flancs 11a, 11b vérifie la condition suivante :

$$\theta < \pi/2 - \alpha$$

où $\theta$ l'angle de mouillage défini précédemment et $\alpha$ l'angle d'inclinaison du flanc considéré 11a, 11b par rapport au capot 14. Ainsi, cet agencement des flancs du canal en forme de V permet de générer un « effet de pointe » au niveau de l'interface de l'échantillon liquide, ce qui permet d'augmenter la vitesse d'écoulement du liquide dans le canal.

Dans cet exemple, à titre illustratif, la profondeur du canal est de $150\mu m$, la largeur Lmax du canal au niveau du capot 14 est de $1300\mu m$ et la largeur Lmin au niveau de la paroi de fond 10 est de $700\mu m$. On obtient alors un angle d'inclinaison $\alpha$ de 26° environ entre les flancs 11a, 11b et le capot.

Ces exemples d'amincissement du canal en bordure de celui-ci sont donnés à titre illustratif et d'autres agencements (non représentés) sont possibles. Ainsi, en variante de la figure 6, les flancs peuvent être inclinés dans un sens opposé, de manière à former un angle aigu non plus avec la surface du capot mais avec la surface de la paroi de fond. La profondeur en bordure du canal est alors définie par la distance suivant l'axe Z entre le flanc considéré et la paroi de fond. Une autre variante est possible, où les flancs sont structurés de manière à former chacun une forme de V. Plus précisément, le flanc structuré présente deux portions planes inclinées mutuellement, et formant un angle aigu à l'intersection d'une première portion avec la paroi de fond, et un angle aigu à l'intersection de la deuxième portion avec le capot. Le demi-angle formé à l'intersection des deux portions vérifie avantageusement la condition de Concus-Finn.

Dans une autre variante non représentée, la paroi de fond 10 n'est pas plane, mais structurée de manière à présenter une forme en V, en coupe transversale. Plus précisément, la paroi de fond peut être formée de deux portions dont chacune rejoint le flanc correspondant, et où l'intersection des deux portions définie un angle inférieur à 180°, et de manière avantageuse inférieur à 90°.

Dans une autre variante non représentée, les flancs et/ou la paroi de fond présente(nt) une forme courbe, en coupe transversale. Les flancs de forme courbe peuvent est agencés pour former un angle aigu à l'intersection avec le capot qui vérifie avantageusement la condition de Concus-Finn.

L'écoulement capillaire à l'intérieur du canal est obtenu par le fait que l'angle de mouillage de l'échantillon liquide sur la surface interne du canal, au niveau de la ligne triple, est inférieur à 90°. La surface du canal est alors dite hydrophile. Une force motrice de mouillage assure alors l'écoulement du liquide dans le canal. Cette propriété de mouillage de la surface interne du canal peut être obtenue ou renforcée par un traitement chimique de surface, notamment par plasma, par exemple plasma O2, qui permet de diminuer l'angle de mouillage du liquide dans le canal.

La mouillabilité, c'est-à-dire l'angle de mouillage que forme le liquide sur la surface considérée au niveau de la ligne triple, peut être identique pour toute la surface interne du canal. Cependant, la réalisation du dispositif de prélèvement en deux éléments distincts A, B autorise un traitement spécifique de surface des différentes zones ou surfaces internes du canal. Ce traitement de surface est alors réalisé avant insertion de la partie mâle dans la partie femelle, et d'autant plus facilement que le canal présente une section transversale ouverte sur sa partie longitudinale. Ainsi, au moins une portion de la surface de la paroi de fond 10, au moins une portion de la surface des flancs 11a, 11b du canal et/ou au moins une portion de la surface du capot 14 peuvent présenter une mouillabilité différente de la mouillabilité des autres surfaces du canal. En d'autres termes, la surface du capot 14, celle des flancs 11a, 11b et/ou celle de la paroi de fond 10 présente(nt), entièrement ou partiellement, une mouillabilité différente des autres surfaces internes. Ce traitement localisé de la surface du capot 14, de la surface des flancs 11a, 11b et/ou la surface de la paroi de fond 10 du canal permet ainsi de modifier l'angle de mouillage sur la surface traitée et peut ainsi modifier la forme globale de l'interface liquide/gaz notamment au niveau de la ligne triple. Ce traitement spécifique peut alors avoir un effet bénéfique pour notamment éviter la formation de bulle de gaz dans le canal.

Le dispositif, en particulier les parties mâle 9 et femelle 12, peut être réalisé, par exemple, par une technique de moulage ou d'injection d'un matériau plastique tel que du polycarbonate, polypropylène, polyéthylène, cyclo-oléfine-copolymère (COC), cyclo-oléfine-polymère (COP), ou tout autre matériau pouvant convenir.

Pour garantir un moulage et un démoulage efficace du deuxième élément B, il est avantageux de réaliser la partie femelle 14 sous forme conique pour favoriser son démoulage dans un axe unique X. Le deuxième élément est ainsi réalisable dans un moule à un seul axe de démoulage.

Parallèlement, la forme du premier élément A, notamment dans sa partie mâle 9, doit tenir compte de ces formes coniques pour assurer à l'assemblage la mise en contact pour l'herméticité des différentes parois. Cette pièce est aussi réalisable dans un moule à un seul axe de démoulage suivant l'axe Z.

Le matériau formant les parties mâle 9 et femelle 12 est de préférence transparent au rayonnement visible et/ou infrarouge, en particulier lorsqu'une analyse de l'échantillon liquide par des moyens optiques est prévue.

Le dispositif peut présenter une longueur totale, suivant l'axe X, de l'ordre de quelques centimètres, par exemple deux centimètres, la coupelle peut présenter une surface de quelques centimètres carrés, par exemple 2cm x 1cm, et l'ouverture de collecte une longueur de quelques millimètres, par exemple 5mm, sur une largeur de 0,5mm à 1mm. La partie mâle peut présenter une longueur et une largeur de quelques millimètres, par exemple 10mm x 5mm, pour une épaisseur de quelques centaines de microns voire quelques millimètres, par exemple 1mm. Le canal peut présenter une longueur de quelques millimètres ou centimètres, une largeur de quelques centaines de microns à quelques millimètres, et une épaisseur de l'ordre de quelques dizaines de microns à quelques millimètres. A titre d'exemple, la chambre d'analyse peut présenter une longueur (suivant l'axe X) de 6,5mm, une largeur (suivant l'axe Y) de 3mm dans sa zone la plus large, et une profondeur (suivant l'axe Z) de 150$\mu$m. Ces ordres de grandeurs ne sont donnés qu'à titre illustratif.

Le dispositif peut être utilisé pour prélever tout type de liquide, par exemple un liquide biologique éventuellement corporel tel que du sang, de l'urine ou autre. Le dispositif permet de prélever un échantillon du liquide d'intérêt, dans le but notamment de procéder à une analyse chimique et/ou biologique de celui-ci.

Le canal 5, notamment au niveau de sa chambre d'analyse 20, peut être fonctionnalisé par un réactif destiné à réagir avec l'échantillon liquide. Le réactif peut être déposé par un procédé de séchage, qui conduit à un dépôt surfacique dans le canal, ou de lyophilisation, qui conduit à un dépôt volumique.

Pour réaliser le dépôt du réactif, celui-ci est déposé en phase liquide dans la zone voulue du canal 5, par exemple la chambre d'analyse 20, avant toute introduction de la partie mâle 9 dans la partie femelle 12. Dans le cas du séchage, l'évaporation du solvant liquide peut s'effectuer à température ambiante et pression atmosphérique, voire sous vide. Dans le cas de la lyophilisation, on réalise la solidification du solvant contenant le réactif puis la sublimation du solvant. Le réactif lyophilisé se présente alors sous la forme d'un volume poreux apte à se dissoudre au contact de l'échantillon liquide. Il est aussi possible de réaliser une fonctionnalisation biologique ou chimique (ADN, anticorps, protéines, etc...) de la paroi du dispositif, notamment la paroi de fond 10 du canal, par des procédés de couplage chimique avec des liaisons covalentes entre les espèces.

On comprend que la réalisation du dispositif en deux éléments distincts l'un de l'autre permet de simplifier, de raccourcir et de rendre plus précises les étapes de dépôt du réactif sous forme séchée ou lyophilisée. En effet, le dépôt du solvant contenant le réactif peut être effectué directement dans la zone désirée, sans qu'il soit nécessaire de l'introduire par l'entrée du canal pour qu'il migre ensuite jusqu'à la zone d'analyse, comme dans l'exemple de l'art antérieur décrit précédemment. Il est alors possible d'obtenir un dépôt du réactif particulièrement homogène, ce qui est un facteur de qualité pour l'analyse de l'échantillon effectuée ultérieurement ou bien de localiser le réactif à un endroit choisi de la fluidique, ou bien de localiser plusieurs réactifs distincts en des lieux distincts du canal fluidique suivant le besoin de réaliser une réaction de mélange de plus d'un réactif. Les étapes d'évaporation ou de sublimation du solvant sont rendues également plus rapides dans la mesure où la surface d'évaporation ou de sublimation est augmentée par le fait que la section transversale du canal est ouverte sur toute une partie longitudinale, et non pas transversale comme dans l'exemple de l'art antérieur.

Le dispositif peut être utilisé comme un consommable jetable, autorisant une analyse de l'échantillon par des moyens appropriés, réalisée éventuellement en-dehors d'un laboratoire d'analyse.

Les moyens d'analyse peuvent être basés sur une analyse du signal optique émis par l'échantillon liquide ou modifié par celui-ci, les parois des parties mâle et femelle autorisant la transmission du signal optique à mesurer, et éventuellement la transmission d'un signal d'excitation émis par les moyens d'analyse en direction de l'échantillon liquide. A titre d'exemple, la chambre d'analyse peut être soumise à un signal lumineux d'excitation et un capteur optique est disposé de manière à détecter un signal lumineux émis par l'échantillon liquide en réponse à l'absorption du signal d'excitation ou un signal optique modifié par la transmission de l'échantillon liquide. Les longueurs d'onde des signaux optiques peuvent être dans la gamme du visible et/ou de l'infrarouge, et les parties mâle et femelle sont réalisées en un matériau transparent à ces longueurs d'onde.

Les moyens d'analyse peuvent également être basés sur une analyse électrique de l'échantillon liquide, et peuvent comporter une ou plusieurs électrodes disposées dans la chambre d'analyse du canal et reliés électriquement à une source de tension.

Un mode de réalisation des électrodes par dépôt en couche mince des électrodes ainsi que des pistes de contact permettant d'amener la reprise de contact sur le talon du premier élément A peut être utilisé.

La figure 7 est une vue de face d'un dispositif de prélèvement selon un autre mode de réalisation, dans lequel l'évent de mise à l'air libre débouche au niveau de la surface de contact.

Sur cette figure, la partie mâle 9 est logée dans la partie femelle 12 de sorte que l'extrémité de la paroi périphérique 13 de la partie femelle 12 soit en butée contre la surface 24 du talon 23 et la paroi transversale d'extrémité 17 de la partie mâle 9 débouche au niveau de la surface de contact 2.

Le canal 5 comporte une première portion qui s'étend à partir de l'extrémité d'entrée 4 et une chambre d'analyse 20, de largeur supérieure à celle de la première portion, située dans la continuité de la première portion.

Au niveau de la deuxième extrémité 8 du canal 5, un évent 21 de mise à l'air libre communique avec la chambre d'analyse 20 par l'intermédiaire d'un conduit dont la largeur est inférieure à celle de la chambre d'analyse 20 et à celle de l'évent 21. Ce conduit assure la fonction d'arrêt d'écoulement du liquide, par sa différence de largeur avec celle de l'évent.

L'évent 21 prend la forme d'une rainure pratiquée au niveau de la face supérieure de la partie mâle 9, qui s'étend à partir du conduit jusqu'à déboucher au niveau de la surface de contact 2. Ce mode de réalisation permet d'éviter d'avoir à pratiquer une ouverture débouchante au niveau de la paroi périphérique 13 de la partie femelle 12.

Dans une variante non représentée, l'évent peut se présenter sous la forme d'une rainure pratiquée au niveau de la face inférieure de la partie mâle, le conduit de communication fluidique avec la chambre d'analyse s'étendant dans l'épaisseur de la partie mâle. La paroi périphérique de la partie femelle comporte une partie qui forme un capot fermant la section transversale ouverte de l'évent.

La figure 8 est une vue de face d'un dispositif de prélèvement dont la partie mâle est introduite dans la partie femelle, selon un mode de réalisation où une chambre d'analyse 20 du canal, disposée au niveau de la face inférieure de la partie mâle 9, opposée à la face supérieure, comporte une membrane absorbante 27, destinée à être imprégnée par l'échantillon liquide.

Dans cet exemple, le canal 5 comporte une première portion qui s'étend à partir de l'extrémité d'entrée 4, au niveau de la face supérieure de la partie mâle 9, et une chambre d'analyse 20, de largeur supérieure à celle de la première portion, située dans la continuité fluidique de la première portion, et située au niveau de la face inférieure de la partie mâle.

La première portion et la chambre d'analyse communiquent par un conduit (non représenté) s'étendant suivant l'épaisseur de la partie mâle. De préférence, le conduit débouche dans une zone centrale de la chambre d'analyse 20.

Un évent 21 de mise à l'air libre du canal est prévu et s'étend entre la chambre d'analyse 20 et, dans cet exemple, la surface de contact 2, mais il pourrait déboucher au niveau d'une bordure longitudinale de la partie mâle 9, la paroi périphérique 13 de la partie femelle 12 comportant alors une ouverture traversante située en regard de l'orifice de l'évent 21.

La paroi périphérique 13 de la partie femelle 12 comporte une partie qui forme un capot fermant la section transversale ouverte de la chambre d'analyse, de manière à assurer le confinement du canal.

La chambre d'analyse 20 présente ici une forme, dans le plan (X,Y), sensiblement circulaire, mais tout autre forme peut convenir. Elle comporte une membrane 27 apte à absorber l'échantillon liquide par imprégnation à partir d'une face de la membrane parallèle au plan (X,Y) au niveau de laquelle débouche le conduit du canal 5. Ainsi, on obtient une imprégnation essentiellement verticale de la membrane 27 par l'échantillon liquide qui est plus rapide et homogène qu'une imprégnation longitudinale résultant du cas où la membrane est située dans le même plan que la première portion du canal.

Cette membrane peut être constituée d'un empilement de plusieurs matériaux poreux permettant d'assurer des fonctions de filtrage, de pompage, de mélange et/ou d'analyse par migration de l'échantillon biologique par capillarité (ou imprégnation) à travers cette membrane.

Les figures 9a à 9d sont des représentations schématiques, en coupe longitudinale, d'une partie du dispositif de prélèvement représenté sur la figure 8, pour différents moments de l'écoulement de l'échantillon liquide dans le canal.

Dans cet exemple, la chambre d'analyse 20 est disposée au niveau d'une face inférieure 28, opposée à la face supérieure 16 de la partie mâle. La partie femelle comporte une partie de sa paroi périphérique qui forme un second capot 30, celui-ci, au niveau de sa face interne, ferme la section transversale de la chambre d'analyse 20.

L'échantillon liquide est introduit dans la première portion du canal 5 représenté ici en lignes pointillées à partir de l'extrémité d'entrée 4 (figure 9a) et s'écoule jusqu'au conduit 29 de communication fluidique avec la chambre d'analyse (figure 9b). Par souci de clarté, le conduit 29 est représenté ici par une rupture de la ligne pointillée illustrant la paroi de fond alors qu'il est formé d'une portion de canal s'étendant suivant l'épaisseur du canal, suivant l'axe Z du repère représenté sur la figure 8. L'échantillon de liquide s'écoule au travers du conduit 29 jusqu'à la chambre d'analyse 20 et vient au contact de la partie centrale de la membrane 27, ce qui provoque l'imprégnation de celle-ci par l'échantillon liquide (figure 9c). L'imprégnation se fait dans l'épaisseur de la membrane 27 et se poursuit jusqu'à la bordure circulaire de la membrane 27. Lorsque l'imprégnation de la membrane est terminée (figure 9d), une analyse de l'échantillon liquide peut alors être réalisée, par exemple par des moyens optiques aptes à détecter un changement de couleur de la membrane ou de la face inférieure de la membrane.

Le procédé de réalisation d'un dispositif de prélèvement selon l'un des modes de réalisation qui viennent d'être décrits comprend les étapes dans lesquels :

- on réalise un premier élément A comprenant une partie mâle 9, celle-ci comportant au moins un canal 5 à section transversale ouverte, s'étendant longitudinalement entre une première extrémité d'entrée 4 et une seconde extrémité 8, le canal 5 étant formé par une paroi longitudinale 10 de fond de canal bordée de deux parois latérales 11a, 11b formant les flancs du canal 5 ;
- on réalise un second élément B, distinct du premier élément A, comprenant une partie femelle 12, celle-ci comportant une paroi périphérique 13 qui délimite transversalement une cavité destinée à loger la partie mâle 9, une partie 14 de la paroi périphérique 13 étant destinée, lorsque la partie femelle 12 loge la partie mâle 9, à former un capot fermant la section transversale du canal 5,
- on fonctionnalise une zone du canal 5 formant une chambre d'analyse 20 ;
- on introduit la partie mâle 9 dans la partie femelle 12.

Par fonctionnalisation d'une surface, on entend ici le dépôt, sur la surface, d'au moins un élément destiné à interagir avec l'échantillon liquide, par exemple au moins un réactif séché ou lyophilisé, ou l'immobilisation par liaison chimique covalente d'espèces biologiques ou chimique, au moins une électrode, au moins une membrane absorbante.

Le procédé d'analyse d'un échantillon liquide est réalisé à l'aide d'un dispositif de prélèvement selon l'un des modes de réalisation qui viennent d'être décrits. Il comporte les étapes dans lesquelles :

- on introduit la partie mâle 9 dans la partie femelle 12 de sorte que le capot 14 de la partie femelle 12 ferme la section transversale du canal 5 ;
- on dépose un échantillon liquide au niveau de première extrémité d'entrée 4 du canal 5, de sorte que l'échantillon liquide s'écoule par capillarité jusqu'à une chambre d'analyse 20 du canal ;
- on analyse l'échantillon liquide situé dans la chambre d'analyse 20.

Préalablement à l'étape d'introduction de la partie mâle dans la partie femelle, au moins une partie de la surface interne du canal est avantageusement fonctionnalisée en déposant un réactif destiné à réagir avec l'échantillon liquide, une membrane absorbante, voire des électrodes.

Bien entendu, l'invention n'est pas limitée aux exemples de réalisation qui viennent d'être décrits. Diverses modifications peuvent y être apportées par l'homme du métier.

A titre d'exemple, le canal peut comporter une portion s'étendant longitudinalement sous forme de serpentin, c'est-à-dire en présentant une succession de coudes de changement de direction.

Le canal peut comporter, communiquant à une première portion qui s'étend à partir de l'extrémité d'entrée, une pluralité de portions reliée toutes à la première portion et s'étendant chaque jusqu'à sa propre chambre d'analyse.

La partie mâle peut comporter plusieurs canaux, s'étendant chacun à partir de sa propre extrémité d'entrée, éventuellement destinée à déboucher au niveau de la même ouverture de collecte de la surface de contact, ou au niveau d'ouvertures de collecte distinctes les unes des autres. Ainsi, au moins un second canal, à section transversale ouverte, est disposé au niveau d'une face inférieure de la plaque opposée à la face supérieure, éventuellement communiquant avec le canal située au niveau de la face supérieure, et dans lequel une partie de la paroi périphérique de la partie femelle est destiné, lorsque la partie femelle loge la partie mâle, à former un second capot fermant la section transversale du second canal.

On décrit maintenant, en référence aux figures 10a-10c, lla-llc, 12a-12b, et 13a-13b une partie mâle 9 selon un autre mode de réalisation, adapté à coopérer avec la partie femelle 12 telle que décrite précédemment.

La figure 10a est une vue en perspective de la partie mâle et les figures 10b et 10c sont respectivement des vues partielles de dessus et de dessous de la partie mâle illustrée sur la figure 10a.

La partie mâle 9 se distingue des exemples décrits précédemment essentiellement en ce que le canal 5 est bordé d'une zone périphérique 31, ou évidement périphérique, dont la profondeur est supérieure à la profondeur centrale Pc du canal 5.

Plus précisément, la plaque 15 de la partie mâle 9 comporte, au niveau de sa face supérieure 16, des parois latérales 32 de délimitation, qui s'étendent à partir de la paroi transversale d'extrémité 17 de la plaque 15 suivant l'axe longitudinal X du canal 5. Ces parois latérales 32 de délimitation sont des portions en saillie vis-à-vis de la face supérieure 16 de la plaque 15. Elles séparent ainsi deux zones de la face supérieure 16 de la plaque 15 :

- une première zone qui forme le canal 5, délimitée par la surface de fond 10 et les faces intérieures 32a (correspondant aux flancs 11a, 11b) des parois latérales 32 de délimitation, de profondeur centrale Pc définie comme la distance entre la surface de fond 10 et un plan passant par les faces supérieures 32b des parois latérales 32 de délimitation, suivant l'axe Z sensiblement orthogonal à la surface de fond 10 ; et

- une zone périphérique 31 qui s'étend au moins en partie autour du canal 5, délimitée par la face supérieure 16 de la plaque 15 hors de la surface de fond 10, et par les faces extérieures 32c (opposées aux faces intérieures 32a) des parois latérales 32 de délimitation, cette zone périphérique formant un évidement périphérique d'une profondeur Pp supérieure à la profondeur centrale Pc du canal 5.

Ainsi, la face supérieure 16 de la plaque 15 comporte une surface de fond 10 du canal 5 et une surface périphérique 33 de la zone périphérique 31, séparées entre elles par les parois latérales 32 de délimitation en saillie vis-à-vis de la face supérieure 16, la surface périphérique 33 formant un décrochement vis-à-vis de la surface de fond 10 de manière à ce que la zone périphérique 31 forme un évidement d'une profondeur supérieure à la profondeur centrale Pc du canal 5.

Comme le montre la figure 11a, chaque paroi latérale 32 de délimitation comporte une face supérieure 32b, destinée à être au contact du capot 14 lorsque la partie femelle 12 loge la partie mâle 9, la face supérieure 32b reliant les faces intérieure 32a et extérieure 32b. La face intérieure 32a de chaque paroi latérale 32 de délimitation est de préférence inclinée vis-à-vis de la surface de fond 10, de manière à former un angle aigu avec le capot 14, inférieur à 45°, par exemple de 30°, et la face extérieure 32c présente une orientation vis-à-vis du capot 14 de manière à former un angle supérieur à l'angle aigu de la face intérieure 32a, de préférence supérieur ou égal à 60°, et de préférence de l'ordre de 90° de manière à former une variation brusque de la profondeur au niveau de la zone périphérique.

Comme le montre la figure 10b, les parois latérales 32 de délimitation sont distantes l'une de l'autre au niveau de la face d'extrémité 17 de la plaque 15 pour former ensemble l'entrée 4 du canal 5, s'étendent ensuite suivant l'axe longitudinal X du canal 5, puis se rapprochent l'une de l'autre au niveau de l'extrémité 8 de bout du canal 5 pour un former un conduit 34 d'évacuation du gaz éventuellement contenu initialement dans le canal 5 (décrit en détail plus loin, en référence aux figures 13a et 13b) qui autorise la communication fluidique entre le canal 5 et la zone périphérique 31.

A titre purement illustratif, la profondeur centrale Pc du canal 5 peut être de 150 $\mu$m environ alors que la profondeur Pp de la zone périphérique 31 est de 180 $\mu$m environ. Les faces intérieures 32a peuvent être inclinées de 30° environ vis-à-vis du capot. Elles peuvent être espacées de 0,70mm environ au niveau de l'entrée 4 du canal 5, de 1,5mm environ au milieu du canal, et de 0,25mm environ au niveau de l'extrémité 8 de bout du canal 5.

Dans cet exemple, la zone périphérique 31 s'étend au niveau de la face supérieure 16 de la plaque 15, mais également au niveau de la face inférieure 28 et des bordures longitudinales 22. Pour garder une profondeur sensiblement constante de la zone périphérique, des espaceurs sont disposés au niveau de la face inférieure 28 et des bordures longitudinales 22.

Ainsi, des espaceurs 35 sont disposés au niveau des bordures longitudinales 22, sous forme de plots d'espacement en saillie vis-à-vis de chacune de ces bordures 22. Ils maintiennent un espacement sensiblement constant entre la face en regard de la paroi périphérique 13 de la partie femelle 12 et chacune des bordures longitudinales 22, cet espacement ayant une valeur supérieure à la profondeur centrale du canal 5, par exemple une valeur sensiblement égale à la profondeur Pp.

Comme le montre la figure 10c, des espaceurs 36 sont également disposés au niveau de la face inférieure 28 de la plaque 15, sous forme de rails ou parois d'espacement en saillie vis-à-vis de cette face inférieure 28. Ils maintiennent également un espacement sensiblement constant entre la face en regard de la paroi périphérique 13 de la partie femelle 12 et la face inférieure 28, cet espacement ayant une valeur supérieure à la profondeur centrale du canal 5, par exemple une valeur sensiblement égale à la profondeur Pp.

La figure 11a et 11b sont des vues schématiques en coupe transversale d'une partie du dispositif de collecte lorsque la partie femelle 12 loge la partie mâle 9, au niveau du canal 5, lorsque les parois 32 de délimitation sont au contact du capot 14 (fig.11a) et lorsqu'une perte locale de contact a lieu entre les parois 32 de délimitation du canal 5 et le capot 14 (fig.11b).

Sur la figure 11a, la partie mâle 9 est entourée par la paroi périphérique 13 de la partie femelle 12. La face supérieure 16 est structurée de manière à comporter la surface de fond 10 du canal 5 et une surface périphérique 33 de la zone périphérique 31, ces surfaces 10, 33 étant séparées l'une de l'autre par les parois latérales 32 de délimitation. La surface périphérique 33 forme un décrochement vis-à-vis de la surface de fond 10 du canal 5 de manière à former un évidement périphérique 31 d'une profondeur Pp supérieure à la profondeur centrale Pc du canal 5.

Les inventeurs ont ainsi mis en évidence que cette configuration où le canal 5 est entouré au moins en partie par une zone périphérique 31 de plus grande profondeur permet de limiter les fuites de liquide hors du canal 5.

En effet, comme le montre la figure 11b, lorsqu'une perte locale de contact a lieu entre le capot 14 et une paroi de délimitation 32 du canal 5, une fuite de liquide est susceptible d'avoir lieu. Cependant, lorsque le liquide s'écoule par capillarité hors du canal 5 et rejoint la zone périphérique 31, du fait de la profondeur Pp supérieure à la profondeur centrale Pc du canal 5, les forces motrices de capillarité présentent une intensité inférieure à celle des forces de capillarité dans le canal 5. Ainsi, le liquide s'écoule de manière prépondérante le long du canal 5 et non pas hors du canal dans la zone périphérique 31. On assure ainsi un remplissage du canal 5 de manière prépondérante tout en limitant les fuites hors du canal 5.

De plus, comme le montre la figure 11c qui est une vue en détail de la partie en pointillé de la figure 11b, de manière à éviter que du liquide ne s'écoule hors du canal 5 dans la zone périphérique 31 à partir d'une perte locale de contact mécanique entre le capot 14 et la face supérieure 32b, la paroi de délimitation 32 est structurée au niveau de sa face extérieure 32c pour présenter, d'une part une arête vive à l'intersection entre sa face supérieure 32b et sa face extérieure 32c, et d'autre part une augmentation brusque de la profondeur locale. Une arête vive est une ligne d'intersection entre deux plans au niveau de laquelle un angle peut être défini. L'augmentation brusque peut être obtenue par une inclinaison de la face extérieure 32c vis-à-vis du capot 14 pour former un angle supérieur à l'angle d'inclinaison de la face intérieure 32a vis-à-vis du capot. Dans cet exemple, la face intérieure 32a forme un angle de 30° avec le capot 14 alors que la face extérieure 32c forme un angle de 90° environ. Du fait de cette structuration, la ligne triple du liquide en contact avec la paroi 32 de délimitation se retrouve bloquée au niveau de l'arête vive, d'autant que les forces motrices capillaires présentent localement, au niveau de l'évidement périphérique 31, une intensité fortement diminuée du fait de l'augmentation brusque de la profondeur locale. Ainsi, le liquide est piégé au niveau de l'espace local entre la paroi 32 de délimitation et le capot 14 et ne s'écoule pas hors du canal 5.

Les figures 12a et 12b sont des vues schématiques en coupe longitudinale d'une partie du dispositif de collecte lorsque la partie femelle 12 loge la partie mâle 9, au niveau de l'entrée 4 du canal 5 et de la coupelle de collecte 2, lorsqu'une paroi transversale 37 d'étanchéité est en contact avec la paroi périphérique 13 (fig.12a) et lorsqu'une perte locale de contact a lieu entre la paroi transversale 37 d'étanchéité et la paroi périphérique 13 (fig.12b).

Comme le montrent la figure 12a, en lien avec les figures 10a à 10c, la plaque 15 peut également comporter également une paroi transversale 37 d'étanchéité, située au niveau de la face d'extrémité d'entrée 17, qui s'étend de manière circonférentielle en bordure de la plaque 15. Cette paroi transversale 37 d'étanchéité est en saillie vis-à-vis des faces supérieure 16, inférieure 28 et des bordures latérales 22, hormis à l'entrée 4 du canal 5. Elle est destinée à venir contacter la paroi périphérique 13 et affleurer la surface de collecte de la coupelle de contact 2 lorsque la partie femelle 12 loge la partie mâle 9. La paroi 37 d'étanchéité présente une dimension telle qu'elle vient en contact avec la bordure circonférentielle de l'ouverture de collecte 19, hormis au niveau de l'entrée 4 du canal 5. Ainsi, lorsqu'un échantillon liquide est déposé sur la coupelle de contact 2, il s'introduit dans le canal 5 par l'entrée 4 situé au niveau de l'ouverture de collecte 19. Comme le montre la figure 12b, si une perte locale de contact mécanique a lieu entre la paroi transversale 37 d'étanchéité et la paroi périphérique 13, du liquide est susceptible de s'introduire dans la zone périphérique 31. Cependant, les forces motrices capillaires présentent une intensité supérieure au niveau du canal 5 qu'au niveau de la zone périphérique 31, ce qui se traduit par un écoulement capillaire du liquide prépondérant dans le canal 5 par rapport à la zone périphérique 31.

De plus, de manière à éviter que du liquide ne s'écoule dans la zone périphérique 31 à partir d'une perte locale de contact mécanique entre la paroi périphérique 13 et la paroi transversale 37 d'étanchéité, cette dernière est structurée au niveau de sa face intérieure 37a (orientée vers la zone périphérique 31) pour présenter, d'une part une arête vive à l'intersection entre sa face supérieure 37b et sa face intérieure 37a, et d'autre part une augmentation brusque de la profondeur locale. L'augmentation brusque peut être obtenue par une orientation de la face intérieure 37a vis-à-vis de la paroi périphérique 13 de manière à former un angle supérieur à 45°, de préférence supérieur à 60°, et de préférence proche ou égal à 90° comme illustré sur la figure 12b. Du fait de cette structuration, la ligne triple du liquide en contact avec la paroi d'étanchéité 37 se retrouve bloquée au niveau de l'arête vive, d'autant que les forces motrices capillaires présentent localement, au niveau de la zone périphérique 31, une intensité fortement diminuée du fait de l'augmentation brusque de la profondeur locale. Ainsi, le liquide est piégé au niveau de l'espace local entre la paroi d'étanchéité 37 et la paroi périphérique 13 et ne s'écoule pas dans la zone périphérique 31.

Les figures 13a et 13b sont des vues schématiques de l'extrémité du canal au niveau de l'extrémité de bout du canal 5, en vue de dessus (fig.13a) et en coupe longitudinale (fig.l3b).

Comme le montre la figure 13a, les parois latérales 32 de délimitation se rapprochent l'une de l'autre et se rejoignent au niveau de l'extrémité 8 de bout du canal 5. Elles comportent cependant une structuration locale sous forme d'un conduit 34 assurant une communication fluidique entre le canal 5 et la zone périphérique 31 située en aval du canal 5. Ce conduit présente une fonction d'évent et permet d'évacuation du gaz éventuellement présent initialement dans le canal 5, limitant ainsi les risques de formation de bulles dans le canal 5 lors de l'introduction capillaire de l'échantillon liquide.

Dans cet exemple, le conduit 34 présente un rétrécissement de sa largeur ainsi qu'une diminution de sa profondeur à mesure qu'on s'éloigne du canal 5. Ainsi, au niveau de son extrémité opposée au canal 5, et à titre purement illustratif, le conduit peut présenter une largeur minimale de 70μm environ et une profondeur minimale de 30μm environ.

Comme le montre la figure 13b, un décrochement est prévu entre la surface de fond 10 et la surface périphérique 33, de manière à former d'une part une arête vive et d'autre part une augmentation brusque de la profondeur locale. L'augmentation brusque de la profondeur locale peut être obtenue par une orientation de la face extérieure 32c des parois de délimitation 32 au niveau du conduit 34 vis-à-vis du capot 14 avec un angle supérieur à 45°, de préférence supérieur à 60°, et de préférence proche ou égal à 90° comme illustré sur la figure 13b. Du fait de cette structuration, la ligne triple du liquide en contact avec la surface de fond du conduit se retrouve bloquée au niveau de l'arête vive, d'autant que les forces motrices capillaires présentent localement, au niveau de la zone périphérique 31, une intensité fortement diminuée du fait de l'augmentation brusque de la profondeur locale. Ainsi, le liquide est piégé au niveau de l'extrémité du conduit 34 et ne s'écoule pas dans la zone périphérique 31.

D'une manière générale, le dispositif de prélèvement d'un échantillon liquide par capillarité peut comporter :

- un canal 5 qui s'étend longitudinalement entre une première extrémité d'entrée 4 et une seconde extrémité 8,

    ○ le canal 5 étant bordé latéralement par deux parois latérales 32 de délimitation, et, suivant un axe de profondeur du canal, par une surface longitudinale de fond 10 et une surface formant un capot 14 destinée à être en contact avec les parois latérales 32 de délimitation,

- le canal 5 étant entouré transversalement par une zone 31 dite périphérique délimitée par les parois latérales 32 de délimitation, et suivant ledit axe de profondeur par ladite surface formant le capot 14 et une surface opposée 33 dite périphérique,
- la zone périphérique 31 présentant une profondeur, entre le capot 14 et la surface périphérique 33, supérieure à une profondeur du canal 5.

Par profondeur, on entend la dimension entre la surface formant capot 14 et la surface opposée suivant un axe sensiblement orthogonal auxdites surfaces. La surface opposée est la surface de fond 10 dans le cas du canal 5 ou la surface périphérique 33 dans le cas de la zone périphérique 31.

Chaque paroi latérale 32 de délimitation peut comporter une face dite intérieure 32a orientée vers le canal 5, une face dite extérieure opposée 32c orientée vers la zone périphérique 31, et une face dite supérieure 32b reliant lesdites faces intérieure 32a et extérieure 32c, et destinée à être en contact du capot 14. La face extérieure 32c peut présenter une arête vive vis-à-vis de la face supérieure 32b. Par arête vive, on entend une ligne d'intersection entre les plans passant par les faces extérieure et supérieure, au niveau de laquelle un angle peut être défini.

La face extérieure 32c de chaque paroi latérale 32 de délimitation peut présenter un angle d'inclinaison vis-à-vis du capot 14 supérieure supérieur ou égal à 45°, de préférence supérieur ou égal à 60°, et de préférence sensiblement égal à 90°.

Le canal 5 peut être situé sur une face 16 dite supérieure d'une plaque 15, ladite zone périphérique 31, formant un évidement périphérique, s'étendant sur la face supérieure 16 au moins en partie autour du canal 5. La surface périphérique 33 est ainsi en retrait vis-à-vis de la surface de fond 10. La zone périphérique 31 peut en outre s'étendre au niveau d'une face inférieure 28 opposée à la face supérieure 16 de la plaque 15, et/ou s'étendre en outre au niveau de bordures longitudinales 22 de la plaque 15 reliant la face supérieure 16 et la face inférieure 28.

Au moins un espaceur 36 peut être prévu sur la face inférieure 28, destiné à être au contact de la paroi périphérique 13. Au moins un espaceur 35 peut être prévu au niveau de chaque bordure longitudinale 22, destiné à être au contact de la paroi périphérique 13. Ainsi, lorsque la paroi périphérique 13 est en contact avec l'un des espaceurs 35, 36, la zone périphérique 31 présente localement une profondeur Pp supérieure à une profondeur dite centrale Pc du canal.

La plaque 15 peut comporter une face transversale d'extrémité 17 au niveau de laquelle se situe une entrée 4 du canal 5, l'entrée 4 étant située au niveau d'une ouverture de collecte 19 de la paroi périphérique 13. La plaque 15 peut comporter une paroi transversale 37 d'étanchéité qui s'étend de manière en partie circonférentielle en bordure de la face transversale 17 d'extrémité, hormis sur une partie de la face supérieure 16 de manière à former l'entrée 4 du canal 5. La paroi transversale 37 d'étanchéité peut présenter une dimension, suivant un axe perpendiculaire à une face supérieure 16 ou inférieure 28 de la plaque 15, supérieure à une profondeur dite centrale du canal 5.

Le canal 5 peut comporter, au niveau de sa deuxième extrémité 8, un conduit 34 de communication fluidique entre le canal 5 et la zone périphérique 31. Le conduit 34 peut présenter une diminution de sa profondeur et/ou de sa largeur. Les parois latérales 32 de délimitation peuvent se rejoindre au niveau de la deuxième extrémité 8 et présenter une structuration formant ledit conduit 34. La surface de fond 10 au niveau du conduit 34 peut former une arête vive avec une face extérieure 32c des parois de délimitation 32. Ladite face extérieure 32c relie localement la surface périphérique 33 à la surface de fond 10 au niveau du conduit 34. Elle peut présenter un angle vis-à-vis du capot 14 supérieur ou égal à 45°, de préférence supérieur ou égal à 60° et de préférence de l'ordre de 90°.

## Revendications

1. Dispositif de prélèvement (1) d'un échantillon liquide par capillarité, comportant :

    • un premier élément (A), comprenant une partie mâle (9),

      ◦ la partie mâle (9) comportant au moins un canal (5) à section transversale ouverte, s'étendant longitudinalement entre une première extrémité d'entrée (4) et une seconde extrémité (8), le canal (5) étant formé par une paroi longitudinale (10) de fond de canal bordée de deux parois latérales (11a, 11b) formant les flancs du canal (5) ;

    • un second élément (B), distinct du premier élément (A), comprenant une partie femelle (12),

      ◦ la partie femelle (12) comportant une paroi périphérique (13) qui délimite transversalement une cavité destinée à loger la partie mâle (9) ;
      ◦ une partie (14) de la paroi périphérique (13) étant destinée, lorsque la partie femelle (12) loge la partie mâle (9), à former un capot fermant la section transversale du canal (5).

2. Dispositif de prélèvement selon la revendication 1, dans lequel au moins un des flancs (11a, 11b) du canal (5) étant agencé de sorte que, lorsque la partie femelle (12) loge la partie mâle (9), la distance, dans un plan transversal à l'axe longitudinal du canal (9), entre deux parois (10, 11a, 11b, 14) du canal (5) diminue en direction d'une bordure transversale du canal (5).

3. Dispositif de prélèvement selon la revendication 1 ou 2, dans lequel la partie mâle (9) est formée d'une plaque de section transversale sensiblement rectangulaire, le canal (5) étant disposé au niveau d'une face longitudinale dite supérieure (16) de la plaque formant la partie mâle (9).

4. Dispositif de prélèvement selon la revendication 3, dans lequel le canal (5) comporte au moins une portion (20) dite chambre d'analyse, disposée au niveau de la face supérieure (16) de la partie mâle (9) ou au niveau d'une face dite inférieure (28), opposée à la face supérieure, de la partie mâle (9).

5. Dispositif de prélèvement selon la revendication 4, dans lequel le canal (5) comporte au moins une chambre d'analyse (20), à section transversale ouverte, disposée au niveau de la face inférieure (28) de la partie mâle (9), communiquant avec la portion du canal (5) située au niveau de la face supérieure (16), et dans lequel une partie de la paroi périphérique (13) de la partie femelle (12) est destinée, lorsque la partie femelle loge la partie mâle, à former un second capot (30) fermant la section transversale de ladite chambre d'analyse (20).

6. Dispositif de prélèvement selon la revendication 5, dans lequel ladite chambre d'analyse (20) communique avec la portion du canal (5) située au niveau de la face supérieure (16) par un conduit (29) débouchant au niveau d'une zone centrale de ladite chambre d'analyse (20).

7. Dispositif de prélèvement selon l'une quelconque des revendications 3 à 6, dans lequel les dimensions de la partie mâle (9) et celles de la partie femelle (12) définissant la cavité sont choisies de sorte que, lorsque la partie femelle loge la partie mâle, le capot (14, 30) appuie sur la face supérieure (16) de la plaque de la partie mâle (9) ou inversement, et éventuellement sur la face inférieure (28) opposée à la face supérieure (16), de manière à assurer la fermeture hermétique de la section transversale du canal (5).

8. Dispositif de prélèvement selon l'une quelconque des revendications 1 à 7, dans lequel au moins une portion de la surface de la paroi longitudinale de fond (10), au moins une portion des flancs (11a, 11b) du canal et/ou au moins une portion de la surface du capot (14, 30) présente(nt) une mouillabilité différente de celle des autres surfaces du canal (5).

9. Dispositif de prélèvement selon l'une quelconque des revendications 1 à 8, dans lequel le second élément (B) comporte une surface de contact (2) destinée à recevoir l'échantillon liquide, assemblée à la partie femelle (12) de sorte qu'une ouverture de collecte (19) de la cavité débouche au niveau de la surface de contact (2), et dans lequel, lorsque la partie femelle loge à la partie mâle, une paroi transversale d'extrémité (17) de la partie mâle (9) au niveau de laquelle se situe la première extrémité d'entrée (4) du canal (5), affleure la surface de contact (2).

10. Dispositif de prélèvement selon l'une quelconque des revendications 1 à 9, dans lequel la seconde extrémité (8) du canal (5) communique avec un évent (21) débouchant sur l'environnement du dispositif.

11. Dispositif de prélèvement selon l'une quelconque des revendications 1 à 10, dans lequel le premier élément (A) comporte un talon (23) de préhension sur lequel est assemblée la partie mâle (9).

**12.** Dispositif de prélèvement selon la revendication 11, dans lequel le talon (23) est formé d'une plaque qui s'étend dans le plan de la partie mâle (9), et comporte au moins une portion en saillie (25) vis-à-vis de la plaque du talon (23).

**13.** Dispositif de prélèvement selon l'une quelconque des revendications 1 à 12, dans lequel le canal (5) comprend au moins une portion formant une chambre d'analyse (20) comportant des réactifs séchés ou lyophilisés, au moins une électrode, ou une membrane absorbante.

**14.** Dispositif de prélèvement selon l'une quelconque des revendications 1 à 13, dans lequel le canal (5) est délimité transversalement par des parois latérales (32) de délimitation destinées à être en contact avec le capot (14), ces dernières (32) séparant le canal (5) d'une zone périphérique (31) qui entoure au moins en partie le canal (5), le canal (5) comportant une surface dite de fond (10) en regard du capot (14) et la zone périphérique comportant une surface dite périphérique (33) en regard de la paroi périphérique (13), une distance entre la surface périphérique (33) et la paroi périphérique (13) en regard, suivant un axe orthogonal à la surface de fond (10), étant supérieure à une distance entre la surface de fond (10) et le capot (14) en regard.

**15.** Procédé de réalisation d'un échantillon liquide à l'aide d'un dispositif de prélèvement (1) selon l'une quelconque des revendications 1 à 14, comportant les étapes dans lesquelles :

- on réalise un premier élément (A) comprenant une partie mâle (9), celle-ci comportant au moins un canal (5) à section transversale ouverte, s'étendant longitudinalement entre une première extrémité d'entrée (4) et une seconde extrémité (8), le canal (5) étant formé par une paroi longitudinale (10) de fond de canal bordée de deux parois latérales (11a, 11b) formant les flancs du canal (5) ;
- on réalise un second élément (B) distinct du premier élément (A), comprenant une partie femelle (12), celle-ci comportant une paroi périphérique (13) qui délimite transversalement une cavité destinée à loger la partie mâle (9), une partie (14) de la paroi périphérique (13) étant destinée, lorsque la partie femelle (12) loge la partie mâle (9), à former un capot fermant la section transversale du canal (5) ;
- on fonctionnalise une zone du canal (5) formant une chambre d'analyse (20) ;

- on introduit la partie mâle (9) dans la partie femelle (12).

**Patentansprüche**

**1.** Vorrichtung (1) zur Gewinnung einer flüssigen Probe durch Kapillarwirkung, umfassend:

• ein erstes Element (A), umfassend einen männlichen Teil (9),

◦ wobei der männliche Teil (9) mindestens einen Kanal (5) mit offenem Querschnitt umfasst, der sich in Längsrichtung zwischen einem ersten Eintrittsende (4) und einem zweiten Ende (8) erstreckt, wobei der Kanal (5) aus einer Bodenlängswand (10) des Kanals gebildet wird, die von zwei Seitenwänden (11a, 11b) begrenzt wird, welche die Flanken des Kanals (5) bilden;

• ein zweites Element (B), das vom ersten Element (A) verschieden ist, umfassend einen weiblichen Teil (12),

◦ wobei der weibliche Teil (12) eine periphere Wand (13) umfasst, die in Querrichtung einen Hohlraum begrenzt, der dazu bestimmt ist, den männlichen Teil (9) aufzunehmen;
◦ wobei ein Teil (14) der peripheren Wand (13) dazu bestimmt ist, wenn der weibliche Teil (12) den männlichen Teil (9) aufnimmt, eine Abdeckung zu bilden, die den Querschnitt des Kanals (5) verschließt.

**2.** Gewinnungsvorrichtung nach Anspruch 1, wobei mindestens eine der Flanken (11a, 11b) des Kanals (5) derart eingerichtet ist, dass, wenn der weibliche Teil (12) den männlichen Teil (9) aufnimmt, die Distanz, in einer Ebene quer zur Längsachse des Kanals (9), zwischen zwei Wänden (10, 11a, 11b, 14) des Kanals (5) in der Richtung eines Querrands des Kanals (5) abnimmt.

**3.** Gewinnungsvorrichtung nach Anspruch 1 oder 2, wobei der männliche Teil (9) aus einer Platte mit einem im Wesentlichen rechteckigen Querschnitt gebildet ist, wobei der Kanal (5) auf der Höhe einer als obere bezeichneten Längsfläche (16) der Platte angeordnet ist, die den männlichen Teil (9) bildet.

**4.** Gewinnungsvorrichtung nach Anspruch 3, wobei der Kanal (5) mindestens einen Abschnitt (20) umfasst, der als Analysekammer bezeichnet wird, die auf der Höhe der oberen Fläche (16) des männlichen Teils (9) oder auf der Höhe einer als untere bezeichneten Fläche (28), gegenüber der oberen Fläche, des männlichen Teils (9) angeordnet ist.

5. Gewinnungsvorrichtung nach Anspruch 4, wobei der Kanal (5) mindestens eine Analysekammer (20), mit offenem Querschnitt, umfasst, die auf der Höhe der unteren Fläche (28) des männlichen Teils (9) angeordnet ist, und die mit dem Abschnitt des Kanals (5) kommuniziert, der auf der Höhe der oberen Fläche (16) angeordnet ist, und wobei ein Teil der peripheren Wand (13) des weiblichen Teils (12) dazu bestimmt ist, wenn der weibliche Teil den männlichen Teil aufnimmt, eine zweite Abdeckung (30) zu bilden, die den Querschnitt der Analysekammer (20) verschließt.

6. Gewinnungsvorrichtung nach Anspruch 5, wobei die Analysekammer (20) mit dem Abschnitt des Kanals (5), der auf der Höhe der oberen Fläche (16) angeordnet ist, durch eine Leitung (29) kommuniziert, die auf der Höhe einer zentralen Zone der Analysekammer (20) einmündet.

7. Gewinnungsvorrichtung nach einem der Ansprüche 3 bis 6, wobei die Abmessungen des männlichen Teils (9) und jene des weiblichen Teils (12), die den Hohlraum definieren, derart ausgewählt sind, dass, wenn der weibliche Teil den männlichen Teil aufnimmt, die Abdeckung (14, 30) auf der oberen Fläche (16) der Platte des männlichen Teils (9) aufliegt, oder umgekehrt, und gegebenenfalls auf der unteren Fläche (28) gegenüber der oberen Fläche (16), um den hermetischen Verschluss des Querschnitts des Kanals (5) sicherzustellen.

8. Gewinnungsvorrichtung nach einem der Ansprüche 1 bis 7, wobei mindestens ein Abschnitt der Fläche der Bodenlängswand (10), mindestens ein Abschnitt der Flanken (11a, 11b) des Kanals und/oder mindestens ein Abschnitt der Fläche der Abdeckung (14, 30) eine Formbarkeit aufweist (aufweisen), die von jener der anderen Flächen des Kanals (5) verschieden ist.

9. Gewinnungsvorrichtung nach einem der Ansprüche 1 bis 8, wobei das zweite Element (B) eine Kontaktfläche (2) aufweist, die dazu bestimmt ist, die flüssige Probe aufzunehmen, und die mit dem weiblichen Teil (12) derart zusammengefügt ist, dass eine Sammelöffnung (19) des Hohlraums auf der Höhe der Kontaktfläche (2) einmündet, und wobei, wenn der weibliche Teil den männlichen Teil aufnimmt, eine Endquerwand (17) des männlichen Teils (9), auf deren Höhe die erste Eintrittsöffnung (4) des Kanals (5) angeordnet ist, mit der Kontaktfläche (2) fluchtet.

10. Gewinnungsvorrichtung nach einem der Ansprüche 1 bis 9, wobei das zweite Ende (8) des Kanals (5) mit einem Loch (21) kommuniziert, das in der Umgebung der Vorrichtung einmündet.

11. Gewinnungsvorrichtung nach einem der Ansprüche 1 bis 10, wobei das erste Element (A) einen Greifansatz (23) umfasst, an dem der männliche Teil (9) angebracht ist.

12. Gewinnungsvorrichtung nach Anspruch 11, wobei der Ansatz (23) aus einer Platte gebildet ist, die sich in der Ebene des männlichen Teils (9) erstreckt, und mindestens einen vorspringenden Abschnitt (25) in Bezug auf die Platte des Ansatzes (23) umfasst.

13. Gewinnungsvorrichtung nach einem der Ansprüche 1 bis 12, wobei der Kanal (5) mindestens einen Abschnitt umfasst, der eine Analysekammer (20) bildet, die getrocknete oder lyophilisierte Reagenzien, mindestens eine Elektrode oder eine absorbierende Membran umfasst.

14. Gewinnungsvorrichtung nach einem der Ansprüche 1 bis 13, wobei der Kanal (5) in Querrichtung durch Begrenzungsseitenwände (32) begrenzt ist, die dazu bestimmt sind, mit der Abdeckung (14) in Kontakt zu stehen, wobei diese Letzteren (32) den Kanal (5) von einer peripheren Zone (31) trennen, die mindestens teilweise den Kanal (5) umgibt, wobei der Kanal (5) eine als Boden bezeichnete Fläche (10) gegenüber der Abdeckung (14) umfasst, und die periphere Zone eine als peripher bezeichnete Fläche (33) gegenüber der peripheren Wand (13) umfasst, wobei eine Distanz zwischen der peripheren Fläche (33) und der gegenüberliegenden peripheren Wand (13) gemäß einer Achse orthogonal zur Bodenfläche (10) größer ist als eine Distanz zwischen der Bodenfläche (10) und der gegenüberliegenden Abdeckung (14).

15. Verfahren zur Herstellung einer flüssigen Probe mit Hilfe einer Gewinnungsvorrichtung (1) nach einem der Ansprüche 1 bis 14, umfassend die Schritte, in denen:

- ein erstes Element (A) hergestellt wird, umfassend einen männlichen Teil (9), wobei dieser mindestens einen Kanal (5) mit offenem Querschnitt umfasst, der sich in Längsrichtung zwischen einem ersten Eintrittsende (4) und einem zweiten Ende (8) erstreckt, wobei der Kanal (5) aus einer Bodenlängswand (10) des Kanals gebildet wird, die von zwei Seitenwänden (11a, 11b) begrenzt wird, welche die Flanken des Kanals (5) bilden;
- ein zweites Element (B), das vom ersten Element (A) verschieden ist, hergestellt wird, umfassend einen weiblichen Teil (12), wobei dieser eine periphere Wand (13) umfasst, die in Querrichtung einen Hohlraum begrenzt, der dazu bestimmt ist, den männlichen Teil (9) aufzunehmen, wobei ein Teil (14) der peripheren Wand (13) dazu bestimmt ist, wenn der weibliche Teil (12) den männlichen Teil (9) aufnimmt, eine Abdeckung zu bilden, die den Querschnitt des Kanals (5) verschließt;

- eine Zone des Kanals (5) funktionalisiert wird, die eine Analysekammer (20) bildet;
- der männliche Teil (9) in den weiblichen Teil (12) eingeführt wird.

**Claims**

1. Device for collecting (1) a liquid sample by capillarity, comprising:

  • a first element (A), comprising a male part (9),

    ∘ the male part (9) comprising at least one channel (5) with open transverse section, extending longitudinally between a first input end (4) and a second end (8), the channel (5) being formed by a channel-bottom longitudinal wall (10) bordered by two lateral walls (11a, 11b) forming the sides of the channel (5);

  • a second element (B) distinct from the first element (A), comprising a female part (12),

    ∘ the female part (12) comprising a peripheral wall (13) which transversely delimits a cavity intended to house the male part (9);
    ∘ a part (14) of the peripheral wall (13) being intended, when the female part (12) houses the male part (9), to form a cap closing the transverse section of the channel (5).

2. Sample-collecting device according to Claim 1, in which at least one of the sides (11a, 11b) of the channel (5) being arranged such that, when the female part (12) houses the male part (9), the distance, in a plane transversal to the longitudinal axis of the channel (9), between two walls (10, 11a, 11b, 14) of the channel (5) decreases towards a transverse edge of the channel (5).

3. Sample-collecting device according to Claim 1 or 2, in which the male part (9) is formed by a plate of substantially rectangular transverse section, the channel (5) being disposed at the level of a so-called top longitudinal face (16) of the plate forming the male part (9).

4. Sample-collecting device according to Claim 3, in which the channel (5) comprises at least one portion (20) called analysis chamber, disposed at the level of the top face (16) of the male part (9) or at the level of a so-called bottom face (28), opposite the top face, of the male part (9).

5. Sample-collecting device according to Claim 4, in which the channel (5) comprises at least one analysis chamber (20), with open transverse section, disposed at the level of the bottom face (28) of the male part (9), communicating with the portion of the channel (5) situated at the level of the top face (16), and in which a part of the peripheral wall (13) of the female part (12) is intended, when the female part houses the male part, to form a second cap (30) closing the transverse section of said analysis chamber (20).

6. Sample-collecting device according to Claim 5, in which said analysis chamber (20) communicates with the portion of the channel (5) situated at the level of the top face (16) by a duct (29) emerging at the level of a central zone of said analysis chamber (20).

7. Sample-collecting device according to any one of Claims 3 to 6, in which the dimensions of the male part (9) and those of the female part (12) defining the cavity are chosen such that, when the female part houses the male part, the cap (14, 30) bears on the top face (16) of the plate of the male part (9) or vice versa, and possibly on the bottom face (28) opposite the top face (16), so as to ensure the hermetic sealing of the transverse section of the channel (5).

8. Sample-collecting device according to any one of Claims 1 to 7, in which at least a portion of the surface of the bottom longitudinal wall (10), at least a portion of the sides (11a, 11b) of the channel and/or at least a portion of the surface of the cap (14, 30) exhibits (exhibit) a wettability different from that of the other surfaces of the channel (5).

9. Sample-collecting device according to any one of Claims 1 to 8, in which the second element (B) comprises a contact surface (2) intended to receive the liquid sample, assembled with the female part (12) such that a collection opening (19) of the cavity emerges at the level of the contact surface (2), and in which, when the female part houses the male part, an end transverse wall (17) of the male part (9) at the level of which is located the first input end (4) of the channel (5), is flush with the contact surface (2).

10. Sample-collecting device according to any one of Claims 1 to 9, in which the second end (8) of the channel (5) communicates with a vent (21) emerging on the environment of the device.

11. Sample-collecting device according to any one of Claims 1 to 10, in which the first element (A) comprises a gripping heel (23) on which the male part (9) is assembled.

12. Sample-collecting device according to Claim 11, in which the heel (23) is formed by a plate which extends in the plane of the male part (9), and comprises at least one protruding portion (25) facing the plate of the heel (23).

13. Sample-collecting device according to any one of Claims 1 to 12, in which the channel (5) comprises at least one portion forming an analysis chamber (20) comprising dried or freeze-dried reagents, at least one electrode, or one absorbent membrane.

14. Sample-collecting device according to any one of Claims 1 to 13, in which the channel (5) is delimited transversely by delimiting lateral walls (32) intended to be in contact with the cap (14), the latter (32) segregating the channel (5) from a peripheral zone (31) which at least partly surrounds the channel (5), the channel (5) comprising a so-called bottom surface (10) facing the cap (14) and the peripheral zone comprising a so-called peripheral surface (33) facing the peripheral wall (13), a distance between the peripheral surface (33) and the facing peripheral wall (13), along an axis orthogonal to the bottom surface (10), being greater than a distance between the bottom surface (10) and the facing cap (14).

15. Method for producing a liquid sample using a sample-collecting device (1) according to any one of Claims 1 to 14, comprising the steps of:

- producing a first element (A) comprising a male part (9), the latter comprising at least one channel (5) with open transverse section, extending longitudinally between a first input end (4) and a second end (8), the channel (5) being formed by a channel-bottom longitudinal wall (10) bordered by two lateral walls (11a, 11b) forming the sides of the channel (5);
- producing a second element (B), distinct from the first element (A), comprising a female part (12), the latter comprising a peripheral wall (13) which transversely delimits a cavity intended to house the male part (9), a part (14) of the peripheral wall (13) being intended, when the female part (12) houses the male part (9), to form a cap closing the transverse section of the channel (5);
- functionalizing a zone of the channel (5) forming an analysis chamber (20) is functionalized;
- introducing the male part (9) into the female part (12).

**Fig.1**

**Fig.2**

**Fig.3**

**Fig.4**

Fig.5

Fig.6

17    2

21

13

4

5    20

12    8

9

24

23

26

**Fig.7**

17    2

4

21

5

27

20

12

9

13

X

Y    Z

23

**Fig.8**

26

**Fig.9a**

**Fig.9b**

**Fig.9c**

**Fig.9d**

**Fig.10a**

**Fig.10b**

**Fig.10c**

**Fig.11a**

**Fig.11b**

**Fig.11c**

**Fig.12a**

**Fig.12b**

**Fig.13a**

**Fig.13b**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2014135652 A

**Littérature non-brevet citée dans la description**

- **A. GLIÈRE ; C. DELATTRE.** *Modeling and fabrication of capillary stop valves for planar microfluidic systems,* 2006, vol. 130-131, 601-608